# EUROPEAN PATENT SPECIFICATION

(11) **EP 2 482 072 B1**
(45) Date of publication and mention of the grant of the patent: **06.07.2016**
(21) Application number: 11152289.2
(22) Date of filing: 26.01.2011
(51) Int. Cl.: G01N 33/533, G01N 33/573, G01N 33/58, C07D 233/00, C07D 333/00, C07D 473/00

(54) **A PHOTOLUMINESCENT PROBE**
PHOTOLUMINESZIERENDEN SONDE
SONDE PHOTOLUMINESCENT

(43) Date of publication of application: 01.08.2012
(73) Proprietor: University of Tartu, 50090 Tartu (EE)
(72) Inventor: Uri, Asko, 50606 Tartu (EE); Enkvist, Erki, 50103 Tartu (EE); Vaasa, Angela, 61411 Tartumaa (EE); Kasari, Marje, 50412 Tartu (EE)
(74) Representative: Sagittarius IP

(56) References cited:
- WO-A2-2008/019696
- Angela Vaasa: "Development of fluorescence-based kinetic and binding assays for characterization of protein kinases and their inhibitors.", Dissertation; Institute of Chemistry, Faculty of Science and Technology, University of Tartu, Estonia. , 25 August 2010 (2010-08-25), XP007918964, ISSN: 1406-0299 ISBN: 978-9949-19-405-6 Retrieved from the Internet: URL:https://dspace.utlib.ee/dspace/bitstre am/handle/10062/15125/vaasa_angela.pdf?seq uence=1 [retrieved on 2011-06-27]
- LAVOGINA D ET AL: "Adenosine analogue-oligo-arginine conjugates (ARCs) serve as high-affinity inhibitors and fluorescence probes of type I cGMP-dependent protein kinase (PKGIalpha)", BIOCHIMICA ET BIOPHYSICA ACTA (BBA) - PROTEINS & PROTEOMICS, ELSEVIER, NETHERLANDS, vol. 1804, no. 9, 18 April 2010 (2010-04-18), pages 1857-1868, XP027170139, ISSN: 1570-9639, DOI: DOI:10.1016/J.BBAPAP.2010.04.007 [retrieved on 2010-07-22]
- ENKVIST E ET AL: "Effect of the structure of adenosine mimic of bisubstrate-analog inhibitors on their activity towards basophilic protein kinases", BIOORGANIC & MEDICINAL CHEMISTRY LETTERS, PERGAMON, ELSEVIER SCIENCE, GB, vol. 19, no. 21, 1 November 2009 (2009-11-01), pages 6098-6101, XP026673787, ISSN: 0960-894X, DOI: DOI:10.1016/J.BMCL.2009.09.026 [retrieved on 2009-09-12]
- URI A ET AL: "Bisubstrate fluorescent probes and biosensors in binding assays for HTS of protein kinase inhibitors", BIOCHIMICA ET BIOPHYSICA ACTA (BBA) - PROTEINS & PROTEOMICS, ELSEVIER, NETHERLANDS, vol. 1804, no. 3, 29 October 2009 (2009-10-29), pages 541-546, XP026886287, ISSN: 1570-9639, DOI: DOI:10.1016/J.BBAPAP.2009.10.019 [retrieved on 2009-10-29]
- VAASA A ET AL: "High-affinity bisubstrate probe for fluorescence anisotropy binding/displacement assays with protein kinases PKA and ROCK", ANALYTICAL BIOCHEMISTRY, ACADEMIC PRESS INC, NEW YORK, vol. 385, no. 1, 1 February 2009 (2009-02-01), pages 85-93, XP025838518, ISSN: 0003-2697, DOI: DOI:10.1016/J.AB.2008.10.030 [retrieved on 2008-10-31]
- Erki Enkvist ET AL: "Protein-Induced Long Lifetime Luminescence of Nonmetal Probes", ACS Chemical Biology, vol. 6, no. 10, 21 October 2011 (2011-10-21), pages 1052-1062, XP055019147, ISSN: 1554-8929, DOI: 10.1021/cb200120v
- KASARI MARJE ET AL: "Responsive microsecond-lifetime photoluminescent probes for analysis of protein kinases and their inhibitors", BIOCHIMICA ET BIOPHYSICA ACTA (BBA) - PROTEINS & PROTEOMICS, ELSEVIER, NETHERLANDS, vol. 1834, no. 7, 14 March 2013 (2013-03-14), pages 1330-1335, XP028562776, ISSN: 1570-9639, DOI: 10.1016/J.BBAPAP.2013.02.039
- Ulf Zander ET AL: "On the Phosphorescence of Benzologues of Furan , Thiophene , Selenophene , and Tellurophene. A Systematic Study of the Intra-annular Internal Heavy-atom Effect.", Zeitschrift für Naturforschung A, vol. 44a, 1 January 1989 (1989-01-01), pages 205-209, XP055203069,

## Description

### FIELD OF THE INVENTION

The invention concerns novel photoluminescent probes whose specific association with purine-binding proteins leads to increased emission of long lifetime luminescence, and the application of the probes for monitoring activity of protein kinases (PKs) and other purine-binding proteins, screening of compounds as inhibitors of PKs and characterization of inhibitors targeted to the kinase, and methods of manufacturing of such probes.

The invention also relates to the application of the luminescent probes in biochemical and cellular screening assays for identifying compounds that bind to and modulate the activity of a wide variety of members of PK super-family. The invention relates to the assay for identification and evaluation of PK inhibitors and determination of the concentration of PKs.

Probes of the invention can be used for, for example, monitoring the responsiveness of cells to receptor activation, high throughput screening of inhibitors of PKs and agonists and antagonists of metabotropic and ionotropic receptors, medical diagnosis, tissue imaging.

### BACKGROUND TO THE INVENTION

Altogether ca 3000 proteins (protein kinases, small G proteins, dehydrogenases, ATPases, helicases, non-conventional purine-utilizing proteins, synthtases, purinergic receptors, etc.) are known to possess a purine-binding site, forming the humane purinome. Thus, approximately 13% of protein-coding genes of the human genome are devoted to coding for purine-binding proteins. [Knapp et al., Curr. Top. Med. Chem. 6 (2006) 1129] PKs are the most prevalent of proteins binding a purine nucleotide, ATP. The human genome codes 518 different PKs. [Manning et al., Science 298 (2002) 1912]

### Protein Kinases and Protein Kinase Inhibitors

PKs play a key role in the regulation of protein functions in living cells. The activity of one third of proteins is regulated through phosphorylation of one or more of serine, theorine and thyrosine residues of the protein catalyzed by a PK. More than 400 human diseases (incl. cancer) have been linked to aberrant PK signaling. This has made PK the second largest drug target (and fastest growing category of drugs in development) after G protein-coupled receptors. [Cohen, Nat. Rev. Drug Discov. 1 (2002) 309; Fischer, Curr. Med. Chem. 11 (2004) 1563; Zhang et al., Nat Rev Cancer. 9 (2009) 28] Almost 150 inhibitors targeted to 40 different PKs have reached clinical trials. [Fedorov, Nature Chem. Biol. 6 (2010) 166]

PKs follow ternary complex kinetic mechanism in which direct transfer of the phosphoryl group from ATP to the protein substrate occurs in the active site. [Adams, Chem. Rev. 101 (2001) 2271]

The majority of known inhibitors realize their inhibitory potency via competitive with ATP binding to the purine-binding pocket of PKs. Despite serious selectivity problems (all 518 PKs and more than 2500 other proteins possess a purine-binding site) the main efforts of drug companies have been directed to the development of small-molecule ATP-competitive inhibitors.

Bisubstrate-analog (biligand) inhibitors of PKs also compete with ATP for binding to the PK. These inhibitors additionally associate with the protein binding domain of the enzyme. [Lavogina et al., Chem Med Chem 5 (2010) 23]

### Existing binding assays for PKs

Development of new assay methods for monitoring the activity of PKs and evaluation of inhibitors of PKs has run in parallel with the increase of importance of effective inhibitors for drug industry. [Zaman et al., Comb Chem High Throughput Screen, 6 (2003) 313]

Photoluminescence methods can be spatially and temporally more focused than radioactive methods, and as such, they are more suitable for application in high-throughput screening (HTS) assays. [Olive, Expert Rev Proteomics. 1 (2004) 327] Some small-molecule inhibitors can be conjugated to fluorescent dyes leading to luminescent probes (indicators) that bind to PKs. The term "luminescent probe" as used herein is a compound whose optical (luminescent) properties are changed on binding with a target protein. Usually, the emission intensity, anisotropy and/or lifetime are altered when the analyte (PK) is bound to the probe. The probe can then be displaced by a competitive kinase inhibitor and the change in luminescence characteristics measured to establish the affinity of the non-labeled inhibitor. This interaction therefore forms the basis for a competitive binding assay of kinase inhibitors where neither a substrate nor an antibody to the phosphorylated substrate is required. Several papers and patent applications describe the use of fluorescent probes for determination of the binding characteristics of PK inhibitors. [e.g., Chen et al., 268 (1993) 15812; WO2005/033330].

The importance of binding assays for PKs is quickly increasing [Uri et al., Biochimica et Biophysica Acta 1804 (2010) 541] as small-molecular fluorescent probes can be used for both biochemical assays in kinase solutions and for measurements in living cells.

### Limitations of Current Binding Assays

Both binding and optical properties of luminescent probes have limited their use in binding assays of PKs. Fluorescent probes with micromolar affinity towards PKC were disclosed by Chen et al. [Chen et al., 268 (1993) 15812]. Low (micromolar) affinity and complex fluorescence spectrum make it difficult to use these fluorescent conjugates as probes for binding assays. [WO9906590] Another fluorescent probe described in the patent application WO2005/033330 has affinity in high nanomolar range (K_{d} = 161 nM) which leads to the need for high concentration of the kinase in the assay format (200 nM STK12 kinase was used in the disclosed Example). The requested kinase concentration, arising from the high K_{d} value of the fluorescent probe, is a hundred-fold higher than the kinase concentration usually applied for kinetic measurements (ca 1 nM concentration of the enzyme is often used in kinetic assays). This leads to substantial increase of the cost of the assay. Due to sub-micromolar dissociation constant of the probe it cannot be used for determination of binding constants of inhibitors with nanomolar affinity [Fluorescence Polarization Technical Resource Guide THIRD EDITION 2004, Invitrogen Corporation. Chapter 7]

Bisubstrate inhibitor-based fluorescent probes for PK binding assays with steady-state fluorescence polarization/anisotropy, fluorescence intensity and FRET detection have also been described previously. [WO2008/019696; Vaasa et al., Anal. Biochem 385 (2009) 85; Uri et al., Biochimica et Biophysica Acta 1804 (2010) 541].

The application of long lifetime luminescent labels enables the use of time-gated measurements where the signal is measured with certain delay time after the excitation pulse. This leads to the increase of signal/background ratio. Time-resolved (TR) luminometry (time resolution in micro- or millisecond range) is an excellent measuring regime for homogenous assays because it can free the measured signal from nanosecond-lifetime background fluorescence caused by organic compounds and light scattering. Several recent publications have described the application TR-FRET phenomenon for detection of binding of fluorescent probes to PKs. [Zhang et al., Analytical Biochemistry 343 (2005) 76; Lebakken et al., J Biomol Screen 14 (2009) 924; Kwan et al., Analytical Biochemistry 395 (2009) 256; WO 2010/127980] All these applications use antibodies or other recognition proteins (e.g., avidine, streptavidine) labeled with lanthanide cryptates (or chelates) in combination with fluorescently labeled small-molecule PK inhibitors to get a measurable TR-FRET signal for the complex. In addition to the complexness (both fluorescent probes and antibodies labelled with lanthanide complexes are needed, slow binding of antibodies to the antigen takes time to complete, etc.) and high price of such detection systems, problems with stability of lanthanide complexes in cellular milieu and inability of antibodies to penetrate cell plasma membranes and their instability in cellular milieu make the use of such systems non-applicable for measurements in living cells.

### Room-temperature long lifetime luminescence of small organic molecules

Room-temperature phosphorescence of aromatic compounds in water solution occurs in special conditions. Cyclodextrin-enhanced room-temperature phosphorescence is known for many compounds. [Scypinski et al., Anal. Chem. 56 (1984) 331] Tryptophan phosphorescence of proteins at room temperature has been used as a tool to study protein structure and dynamics. [Papp et al., Photochem. Photobiol. 49 (1989) 775] Room temperature phosphorescence of some aromatic compounds in the liquid state is also a powerful tool in analytical chemistry. [Kuijt et al., Anal. Chim. Acta 488 (2003) 135]

Förster-type of non-radiative dipole-dipole energy transfer [Förster, Ann. Physik. 6 (1948) 55] takes place between two molecules in condition where their energies (emission of donor D with absorption of acceptor A) overlap and they are located at a suitable distance from each other. In addition to the more usual transfer from short lifetime singlet state, radiationless intermolecular resonance energy transfer from long lifetime triplet state of the donor to the singlet state of the acceptor is also in agreement with the Förster dipole-dipole interaction and this phenomen has also been described. [Bennett et al., J. Chem. Phys. 41 (1964) 3040] These data point to the possibility of application of long lifetime luminescent labels as donors for FRET measurements in combination with short lifetime fluorescence acceptors. The substantial enhancement of emission induced by RET from a long-lifetime, low quantum yield donor based on metal-ligand complexes to a long-wavelength, high quantum yield acceptor, has been described and explained. [WO2002/007779; Kang et al., J. Fluoresc. 12 (2002) 97] This phenomenon was visible both inter- and intramolecularly when a ruthenium complex (metal-ligand complex, MLC) with microsecond luminescence lifetime was used as a donor in this system, and a fluorescent red dye BO-PRO-3 was used as the fluorescence acceptor excited by resonance energy transfer. [Maliwal et al., Anal. Chem. 73 (2001) 4277].

Dissertationes Chimicae Universitatis Tartuensis (Angela Vaasa, "Development of fluorescence-based kinetic and binding assays for characterization of protein kinases and their inhibitors") discloses the use of FRET measurements in protein kinase assays to measure the fluorescence properties of the sample, in which time-delay measurements are not used.

Enkvist et al. (ACS Chem. Biol. 6 (2011) 1052) discloses that binding of a thiophene- or selenophene-containing heteroaromatic moiety to the purine-binding pocket of a protein kinase induces a long lifetime photoluminescent signal that is largely intensified through efficient energy transfer to a fluorescent dye present in close proximity to the luminescent donor.

Kasari et al. (Biochim. Biophys. Acta 1834 (2013) 1330) discloses ARC-Lum probes which incorporate thiophene or a selenophene heterocycle and a fluorophore conjugated to the lysine residue in the peptide fragment, which are able to complex with protein kinases.

Zander et al. (Z. Naturforsch 44a (1989) 205) relates to the phosphorescence of benzologues of furan, thiophene, selenophene and tellurophene and in particular, discusses the intra-annular internal heavy atom effect, which determines the photoluminescence properties of many heterocyclic systems.

All previously described methods based on TR-FRET phenomenon use binding proteins labeled with lanthanide cryptates (or chelates) in combination with fluorescently labeled small-molecule PK inhibitors to get a TR-FRET signal for the complex. This make these assays expensive and binding of antibodies require long incubation times. Problems with the stability of metal-ligand complexes in cellular milieu and the inability of antibodies to penetrate cell plasma membranes make the use of such expensive systems non-applicable for measurements in living cells.

### Benefits of the current invention

The current invention concerns small-molecule optical probes whose specific binding to purine-binding site of the target protein leads to substantial change of parameters (e.g., intensity and lifetime) of long lifetime (1-1000 µs) luminescence.

The photoluminescent probes of the current invention are applicable in binding assays for determination of the concentration (activity) of PKs and identification and characterization of inhibitors of PKs. If compared to the probes from previous inventions, the luminescence probes of the present invention are small organic molecules that in complex with a purine-binding protein emit long wavelength light with long lifetime (in microsecond region) that enables the time-gated discrimination over fluorescence present in the sample, either autofluorescence of cells or emission from other fluorophores. A single small-molecule synthetic compound is needed to perform the analysis. The luminescence of the probes in a measurement window after longer delay time (more than 10 µs from the excitation pulse) is substantially increased upon specific binding of the luminescence donor into the purine-binding pocket of the target protein. The novel luminescent probes contain no volumous and degradable metal-ligand complexes that have been previously used as luminescence donors for TR-FRET measurements. The endpoint assays based on the use of luminescent probes of the current invention are very quick, sensitive, and require the use of only one additional chemical (luminescent probe). This makes the assays amenable to automatization and very cost-effective.

### SUMMARY OF THE INVENTION

The invention relates to small-molecule photoluminescent probes comprising a long lifetime luminescence energy donor and a fluorescence energy acceptor for use in binding assays with purine-binding proteins. The non-radiative energy transfer based emission of the acceptor is detected in a suitable time window after delay from donor excitation pulse. The specific binding of the purine-mimicking fragment of the probe to a PK leads to substantial change in the intensity and lifetime of the emitted light. The concentration of the probe bound to a PK in the sample is determined based on the change of the intensity of the emitted light. The probes possess a great gap between the excitation and measurement wavelengths.

The invention also concerns biochemical and cellular bioaffinity assays and tissue imaging with time-resolved luminescence readout based on the use of the probe.

### BRIEF DESCRIPTION OF THE DRAWINGS

Figure 1. Scheme of the kinase induced long lifetime luminescence. LP - long-pass filter, T - luminescence lifetime.
Figure 2. Scheme of the kinase induced long lifetime luminescence in the case of intermolecular resonance energy transfer. LP - long-pass filter T - luminescence lifetime.
Figure 3. General structure of a long-lifetime luminescent probe.
Figure 4. Titration of the luminescent probe ARC-1063 (5 nM) with PKs MSK1 (●), ROCKII (◆), AKT3 (▲), PKA (■), PKG (▼), or PKC (×).
Figure 5. Titration of the luminescent probe ARC-1139 (2 nM) with PKs MSK1 (●), ROCKII (◆), AKT3 (▲), PKA (■), PKG (▼), or PKC (x).
Figure 6. Displacement of the luminescent probe ARC-1063 [at the concentration of 54 nM (■), 18 nM (▲), 6 nM (▼), or 2 nM (◆)] from its complex with PKAc by ARC-902. In the inset the Schild plot analysis of the data is presented (■).
Figure 7. Displacement of fluorescent the luminescent probe ARC-1063 (10 nM) from its complex with ROCK (1 nM) by Fasudil (●), Y27632 (▼), or Staurosporine (▲).
Figure 8. Correlation between the values of inhibition constants (*Kᵢ*) measured in TLC kinetic inhibition assay and displacement constants (*K_{d}*) determined in binding assay using the probe ARC-1063.
Figure 9. Titration of the labeled kinase TAMRA-PKAc with ARC-668. An example of enhancement of luminescence by intermolecular RET.
Figure 10. Increase of long lifetime luminescence intensity that results from the binding of ARC-1063 to the kinase dissociated from complex with ARC-583(Δ), ARC-1041 (■), or Staurosporine(●). Examples of the measurement of the kinetics of dissociation of the inhibitor from its complex with the kinase.
Figure 11. Normalized emission intensity at 675 nm in living C9H6 cells in the presence (■) or absence (□) of ARC-1139. Treatment of the cells with Forskolin (FRSK) leads to dissociation of PKAc from the holoenzyme resulting in the interaction of PKAc with ARC-1139 that leads to increased emission at 675 nm (excitation at 337 nm). The latter effect was reversed by a cell-permeable PKA inhibitor H89.
Figure 12. Activation of PKA by adrenergic agonist isoproterenol in live HEK293 cells as measured through the change of luminescence from ARC-1139.
Figure 13. Activation of PKA by Forskolin in C9H6 cells as measured with ARC-1139 (25 nM) in the cell lysate..

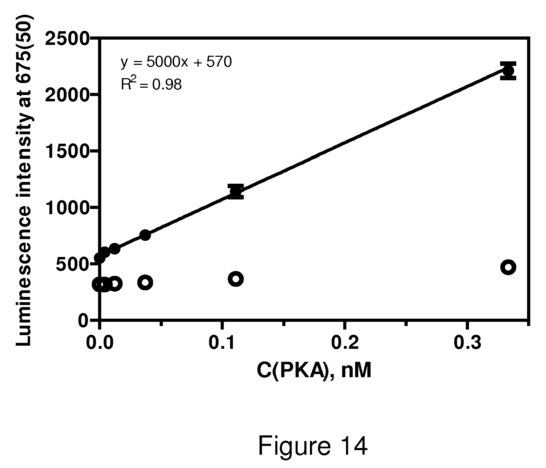

Figure 14. Measurement of low concentrations of the kinase PKAc in human plasma. The luminescence signal of 2 nM ARC-1063 in human blood plasma spiked with concentration range of recombinant PKAc (0-333 pM) in the absence (filled symbols) and presence (empty symbols) of both ATP (100 µM) and PKA selective inhbitor PKI (100 µM). The luminescence signal is directly proportional to the amount of PKA present in the sample. Moreover, the concentration of the endogeneous PKA in blood plasma can be determined from the difference of the results obtained in the presence and absence of PKI. Extracellular PKAc in blood plasma is an acknowledged cancer biomarker. [WO/2000/079281]

### DETAILED DESCRIPTION OF INVENTION

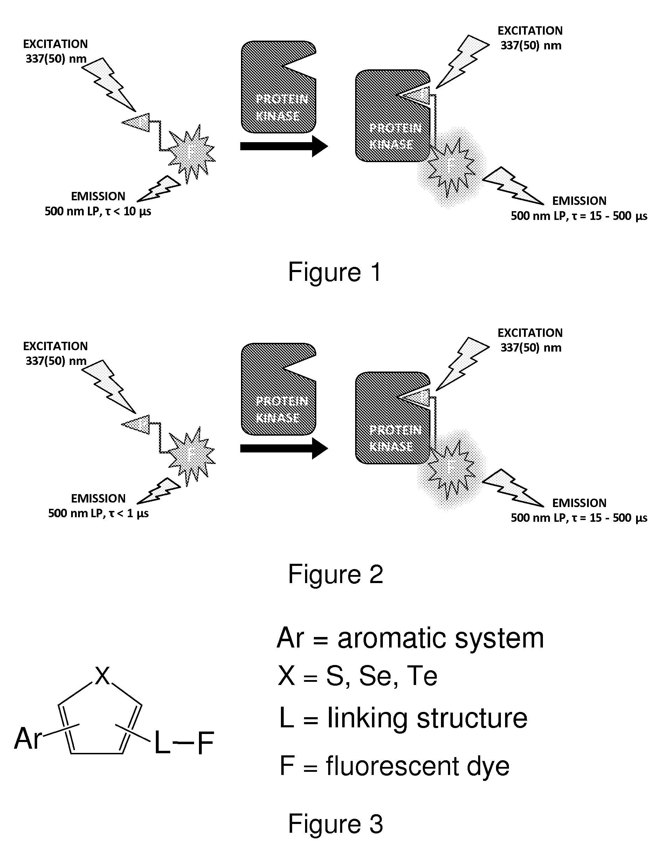

This invention relates to long-lifetime photoluminescence probes whose binding to the target protein induces substantial alteration of the signal intensity emitted by the probe (Fig. 1).

We established and improved molecular structures of luminescent probes whose specific association with the purine-binding site of the target protein leads to substantial change in long lifetime photoluminescence. Our attempt to develop small-molecule long lifetime photoluminescent probes for PKs was based on the following previous knowledge.
1. Phosphorescence of flexible aromatic molecules which are restricted in their excited state reorientation by fixation into a complex with a binding molecule (e.g., cyclodextrin) is largely increased, such binding also reduces collisional quenching of luminescence by oxygen and other impurities.
2. Heavy atoms in aromatic systems have been shown to increase phosphorescence of organic compounds.
3. The possibility of substantial enhancement of emission induced by RET from a long-lifetime, low quantum yield donor to a long-wavelength, high quantum yield acceptor has been previously been shown.

Some organic compounds have shown significant room temperature phosphorescence in micelles, cyclodextrin complexes, polymeric matrixes and crystals. Room temperature phosphorescence originating from the tryptophan residues of a protein has been used to probe structure and molecular mobility of proteins. Restriction of molecular movements is required to diminish nonradiative relaxations of triplet states in all cases of room temperature phosphorescence of pure organic luminophors to produce a detectable signal. Enhancement of luminescence through resonance energy transfer from emitters with low quantum yield to bright energy acceptors has been described. [WO2002/007779]

The current invention provides compounds of the formula P-L-F, wherein P contains a fragment incorporating a long lifetime luminescence donor that binds specifically to a purine-binding pocket of the target protein leading to substantial alteration of the lifetime and intensity of the emitted light when the donor is excited, L is a linker system, and F is a fluorescent dye with good light absorbance and high quantum yield. The most important roles that the linker L has to play in the structure of the luminescent probe P-L-F is the increase of binding affinity of the structural fragment P, tuning of the kinase selectivity of P towards specific PKs, the positioning of the fluorescent label F in a location where it causes minimal hindrance to the binding of the luminescent probe to the kinase, at the same time F should be positioned close to P to achieve maximal energy transfer from the luminescence donor P to the acceptor F. In the case of ATP-competitive inhibitors L may possess a fragment of acknowledged inhibitors whose role in the starting inhibitor is to influence the affinity and selectivity profile of the inhibitor towards PK-s and other purine-binding proteins. P incorporates a fragment of the inhibitor whose optical properties are strongly influenced by its binding to the purine-binding site of the target protein.

A first aspect of the invention is a binding assay method for protein kinases wherein the luminescence of a photoluminescent probe bound to a protein kinase is measured, wherein the probe has structure P-L-F wherein P contains a fragment that has luminescence donor properties and that specifically associates with the purine-binding pocket of the target protein kinase, L is a linker system and F is a fluorescence acceptor dye, said probe containing a heteroaromatic fragment based on a thiophene, selenophene or tellurophene moiety linked to an aromatic system Ar and connected via a linker system L with a fluorescent dye F, said probe having structure as follows: 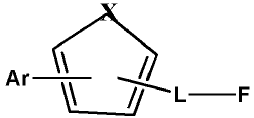
wherein X represents S, Se or Te;
wherein Ar is an aromatic system selected from the list consisting of 2-aminopyrimidine, 2-methylpyrimidine, pyrimidine, pyridine, purine and 7H-pyrrolo[2,3-d]pyrimidine;
wherein the 5-membered S, Se or Te membered ring may additionally optionally incorporate one nitrogen atom;
wherein the 5-membered S, Se or Te containing ring may be optionally substituted by halogeno groups;
wherein the excitation spectrum of the fluorescence acceptor dye F overlaps with the emission spectrum of the luminescent donor P;
comprising (a) exciting said probe bound to the purine-binding pocket of the target protein kinase with a single photon excitation pulse in the wavelength range 280-450 nm and (b) measuring in time-delayed mode luminescence radiation emitted by the probe in the wavelength range 450-1000 nm;
the time-delay being such that the luminescence radiation emitted by the probe is measured free from 1 to 10 nanosecond-lifetime background fluorescence caused by organic compounds and light scattering.

A second aspect of the invention is an in vitro binding assay method for protein kinases in living cells wherein the luminescence of a photoluminescent probe is detected by using a fluorescence microscope, a fluorescence spectrometer or a fluorescence platereader, wherein the probe has structure P-L-F wherein P contains a fragment that has luminescence donor properties and that specifically associates with the purine-binding pocket of the target protein kinase, L is a linker system and F is a fluorescence acceptor dye, said probe containing a heteroaromatic fragment based on a thiophene, selenophene or tellurophene moiety linked to an aromatic system Ar and connected via a linker system L with a fluorescent dye F, said probe having structure as follows: 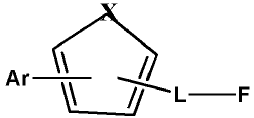
wherein X represents S, Se or Te;
wherein Ar is an aromatic system selected from the list consisting of 2-aminopyrimidine, 2-methylpyrimidine, pyrimidine, pyridine, purine and 7H-pyrrolo[2,3-d]pyrimidine;
wherein L represents 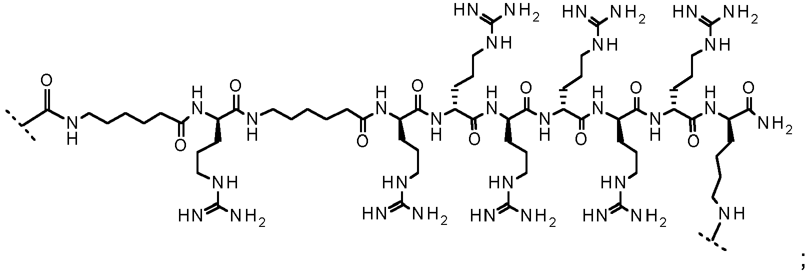
wherein the 5-membered S, Se or Te membered ring may additionally optionally incorporate one nitrogen atom;
wherein the 5-membered S, Se or Te containing ring may be optionally substituted by halogeno groups;
wherein the excitation spectrum of the fluorescence acceptor dye F overlaps with the emission spectrum of the luminescent donor P;
comprising (a) exciting said probe bound to the purine-binding pocket of the target protein kinase with a single photon excitation pulse in the wavelength range 280-450 nm and (b) time-delayed detection of luminescence radiation emitted by the probe in the wavelength range 450-1000 nm;
the time-delay being such that the luminescence radiation emitted by the probe is measured free from 1 to 10 nanosecond-lifetime background fluorescence caused by organic compounds and light scattering.

A third aspect of the invention is use of a photoluminescent probe that has structure P-L-F wherein P contains a fragment that has luminescence donor properties and that specifically associates with the purine-binding pocket of a target protein kinase, L is a linker system and F is a fluorescence acceptor dye, said probe containing a heteroaromatic fragment based on a thiophene, selenophene or tellurophene moiety linked to an aromatic system Ar and connected via a linker system L with a fluorescent dye F, said probe having structure as follows: 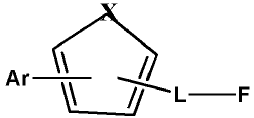
wherein X represents S, Se or Te;
wherein Ar is an aromatic system selected from the list consisting of 2-aminopyrimidine, 2-methylpyrimidine, pyrimidine, pyridine, purine and 7H-pyrrolo[2,3-d]pyrimidine;
wherein the 5-membered S, Se or Te membered ring may additionally optionally incorporate one nitrogen atom;
wherein the 5-membered S, Se or Te containing ring may be optionally substituted by halogeno groups;
wherein the excitation spectrum of the fluorescence acceptor dye F overlaps with the emission spectrum of the luminescent donor P;
in a binding assay for protein kinases;
wherein in the binding assay the luminescence of a photoluminescent probe is measured in time-delayed mode;
wherein said probe, when bound to the purine-binding pocket of the target protein kinase, is excited with a single photon excitation pulse in the wavelength range 280-450 nm and the probe emits luminescence radiation in the wavelength range 450-1000 nm;
the time-delay being such that the luminescence radiation emitted by the probe is measured free from 1 to 10 nanosecond-lifetime background fluorescence caused by organic compounds and light scattering.

A fourth aspect of the invention is use of a photoluminescent probe that has structure P-L-F wherein P contains a fragment that has luminescence donor properties and that specifically associates with the purine-binding pocket of a target protein kinase, L is a linker system and F is a fluorescence acceptor dye, said probe containing a heteroaromatic fragment based on a thiophene, selenophene or tellurophene moiety linked to an aromatic system Ar and connected via a linker system L with a fluorescent dye F, said probe having structure as follows: 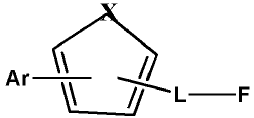
wherein X represents S, Se or Te;
wherein Ar is an aromatic system selected from the list consisting of 2-aminopyrimidine, 2-methylpyrimidine, pyrimidine, pyridine, purine and 7H-pyrrolo[2,3-d]pyrimidine;
wherein the 5-membered S, Se or Te membered ring may additionally optionally incorporate one nitrogen atom;
wherein the 5-membered S, Se or Te containing ring may be optionally substituted by halogeno groups;
wherein the excitation spectrum of the fluorescence acceptor dye F overlaps with the emission spectrum of the luminescent donor P;
wherein the target protein kinase is PKAc;
in a high throughput analysis method for establishment of the level of PKAc activity in a blood plasma sample;
wherein in the method the luminescence of a photoluminescent probe is measured in time-delayed mode;
wherein said probe, when bound to the purine-binding pocket of the target protein kinase, is excited with a single photon excitation pulse in the wavelength range 280-450 nm and the probe emits luminescence radiation in the wavelength range 450-1000 nm;
the time-delay being such that the luminescence radiation emitted by the probe is measured free from 1 to 10 nanosecond-lifetime background fluorescence caused by organic compounds and light scattering.

A fifth aspect of the invention is a photoluminescent probe having structure P-L -F wherein P contains a fragment that has luminescence donor properties and that specifically associates with the purine-binding pocket of the target protein kinase, L is a linker system and F is a fluorescence acceptor dye, said probe containing a heteroaromatic fragment based on a selenophene or tellurophene moiety linked to an aromatic system Ar and connected via a linker system L with a fluorescent dye F, said probe having structure as follows: 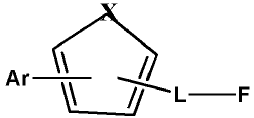
wherein X represents Se or Te;
wherein Ar is an aromatic system selected from the list consisting of 2-aminopyrimidine, 2-methylpyrimidine, pyrimidine, pyridine, purine and 7H-pyrrolo[2,3-d]pyrimidine;
wherein the 5-membered Se or Te membered ring may additionally optionally incorporate one nitrogen atom;
wherein the 5-membered Se or Te containing ring may be optionally substituted by halogeno groups;
wherein the excitation spectrum of the fluorescence acceptor dye F overlaps with the emission spectrum of the luminescent donor P.

More particularly, the probes of use according to aspects of the present invention have the structure 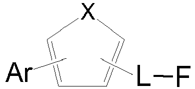
Ar = aromatic system
X = S, Se, Te
L = linking structure
F = fluorescent dye ,
where the aromatic system Ar is an aromatic system selected from the list consisting of 2-aminopyrimidine, 2-methylpyrimidine, pyrimidine, pyridine, purine and 7H-pyrrolo[2,3-d]pyrimidine (FIG.3).

An important unit of the luminescent probes is the heteroaromatic moiety 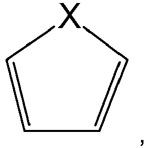 containing as the atom X a sulphur (S, in the case of thiophene), selenium (Se, in the case of selenophene) or tellurium (Te, in the case of tellurophene) atom. Other 5-membered S-, Se- or Te-containing heterocycles, e.g., these that are additionally incorporating 1 nitrogen atom, also possess optical characteristics supporting their use as optical donor moieties of luminescent probes of the present invention. These 5-membered S-, Se- or Te-containing heterocycles may be also substituted with halogeno groups.

The aromatic system Ar incorporates structural units whose role is the probe is the enhancement of binding affinity of the probe to the target protein and the tuning of the binding selectivity towards purine-binding proteins. Ar also tunes the optical properties of the probe making P a long life-time luminescence donor of the probe and rendering the luminescence of the probe sensitive to its binding to the protein. The Ar moiety may be connected to a single atom of the thiophene (selenophene, tellurophene) ring or simultaneously fused to two atoms of the ring while retaining the conjugation between Ar moiety and the thiophene (selenophene, tellurophene) ring. Aromatic systems tested include heteroaromatic systems selected from the list consisting of 2-aminopyrimidine, 2-methylpyrimidine, pyrimidine, pyridine, purine and 7H-pyrrolo[2,3-d]pyrimidine (Table 1).

**Table 1. ARC-codes, structures and HRMS data of the compounds (deconvoluted monoisotopic mass is presented)**

| **ARC-code** | Structure | HRMS Found |
|---|---|---|
| ARC-668 | 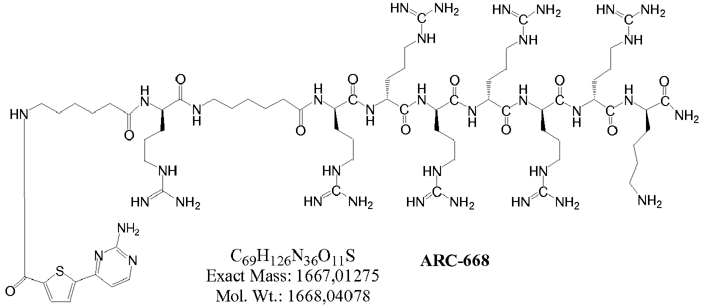 | 1667.01219 |
| ARC-669 | 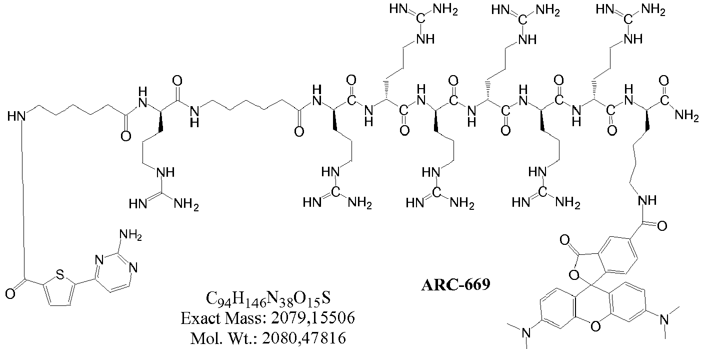 | 2079.15284 |
| ARC-676 | 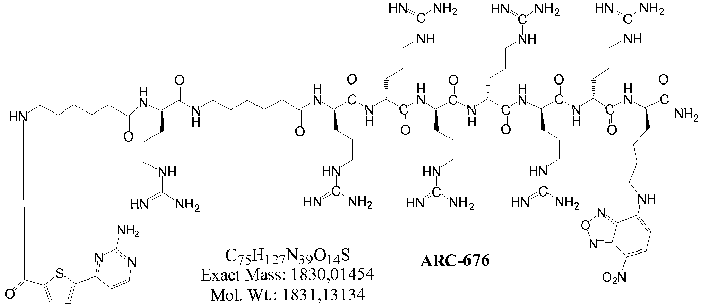 | 1830.01281 |
| ARC-679 | 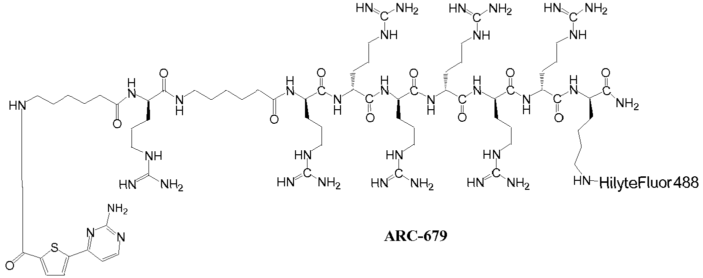 | 2136.1739* |
| ARC-1063 | 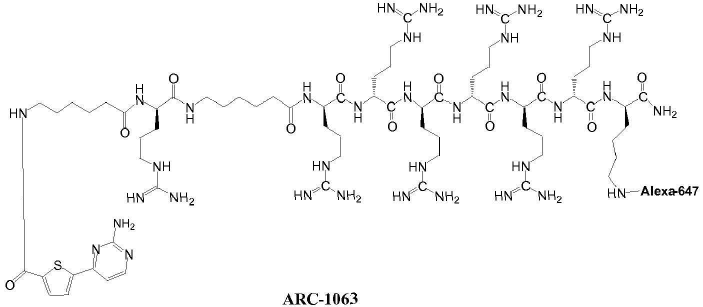 | 2507.1846* |
| ARC-1121 | 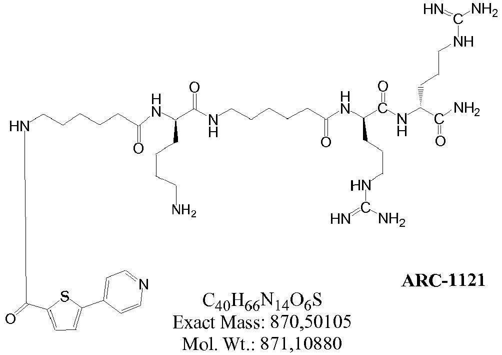 | 870.49849 |
| ARC-1122 | 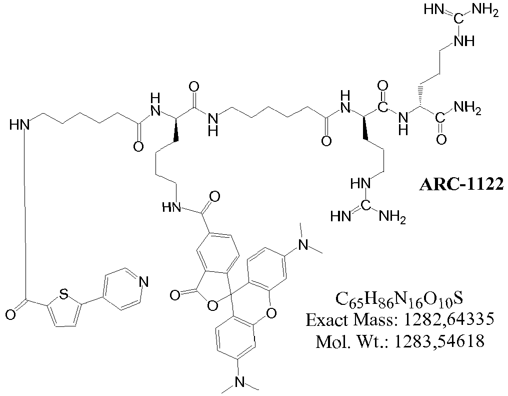 | 1282.64083 |
| ARC-1123 | 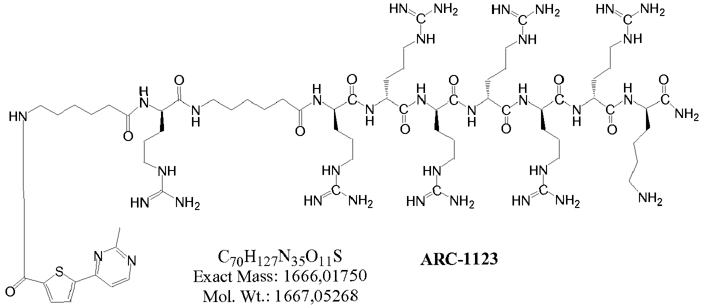 | 1666.01168 |
| ARC-1128 | 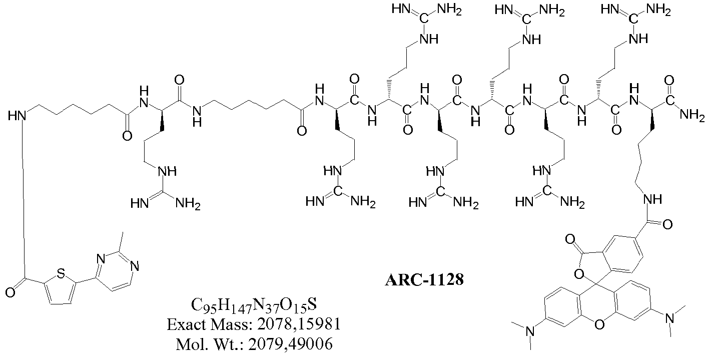 | 2078.16026 |
| ARC-1129 | 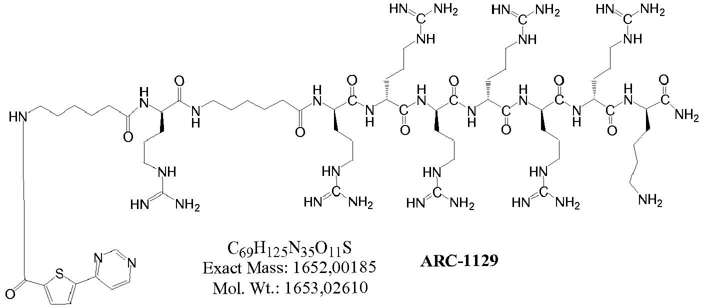 | 1651.99576 |
| ARC-1130 | 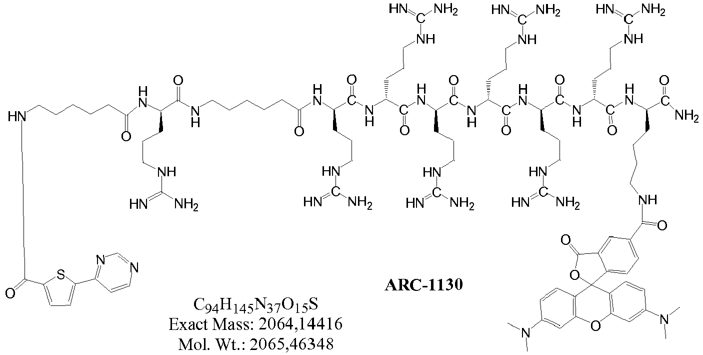 | 2064.14487 |
| ARC-1132 | 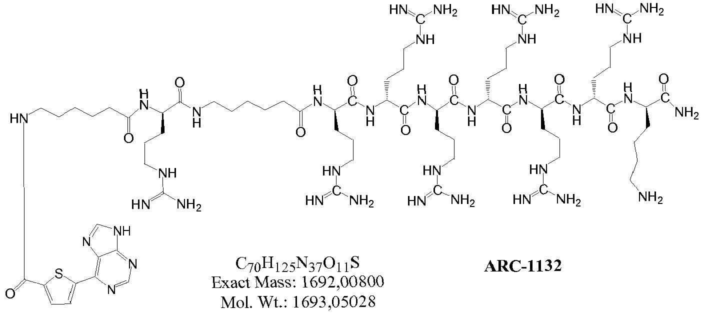 | 1692.01165 |
| ARC-1133 | 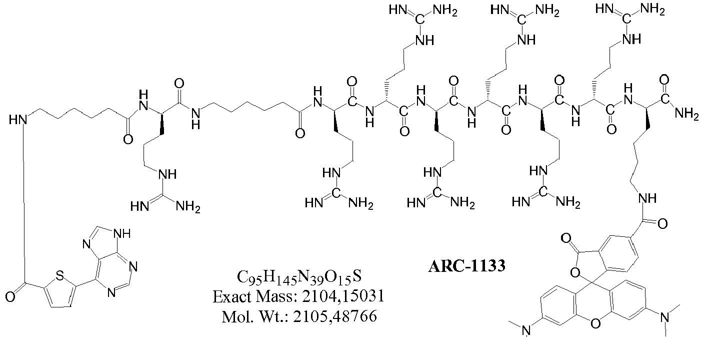 | 2104.15625 |
| ARC-1135 | 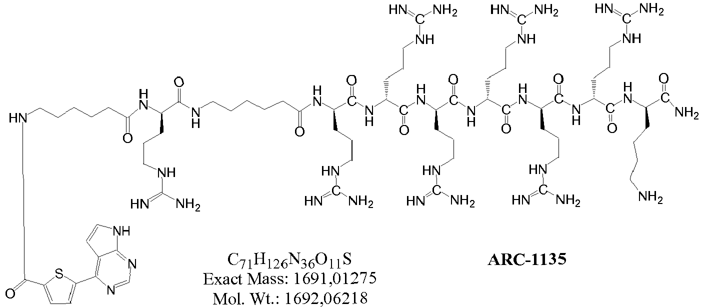 | 1691 |
| ARC-1136 | 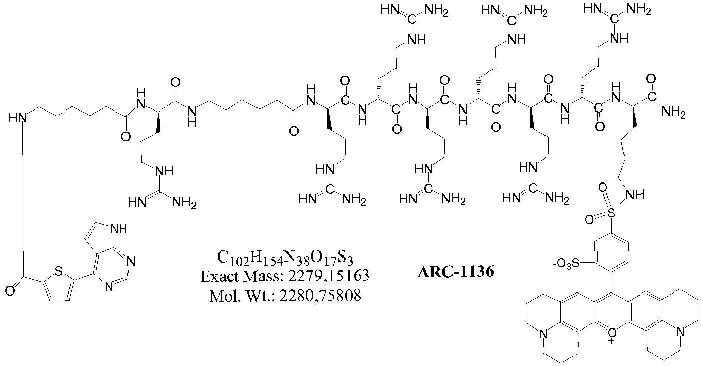 | 2279 |
| ARC-1138 | 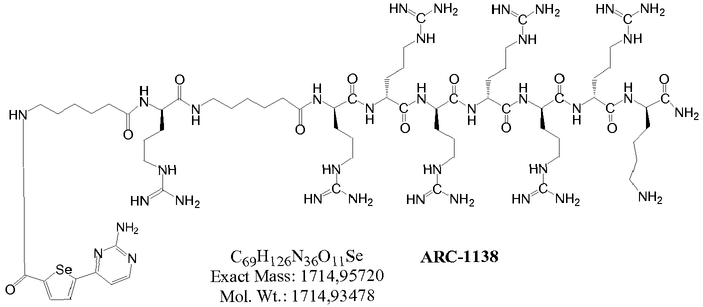 | 1714.95391 |
| ARC-1139 | 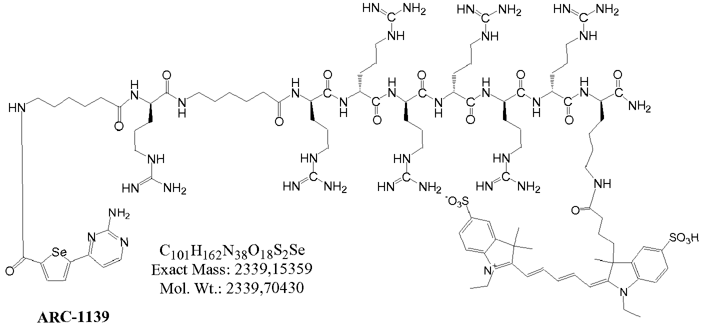 | 2339.14580 |
| ARC-1144 | 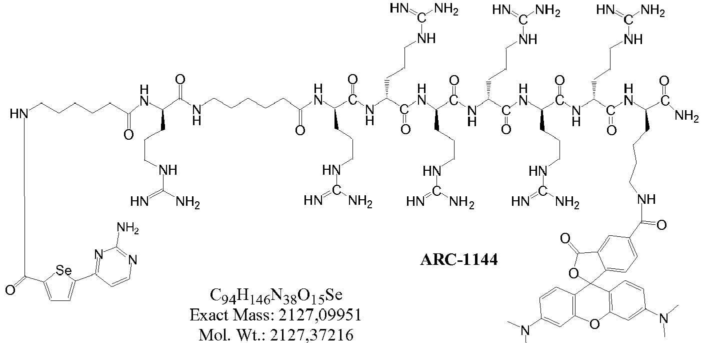 | 2127.09949 |

| | | |
|---|---|---|
| *Structures of HilyteFluor448 and Alexa-647 have not been disclosed. Monoisotopic masses for selenium containing compounds are calculated for most abundant isotope ⁸⁰Se. | | |

Structures of several compounds incorporating P and L moieties but lacking the fluorescent label F are also presented in Table 1 (compounds ARC-668, ARC-676, ARC-1121, ARC-1123, ARC-1129, ARC-1132, ARC-1135, ARC-1138). These compounds with high affinity (subnanomolar or low nanomolar) bind to the catalytic subunit of protein kinase A (PKAc) and some other PKs of the AGC group. They strongly absorb light between 280 - 360 nm (molar absorption coefficients 10000 - 25000 M⁻¹cm⁻¹) and when excited at 337(50) nm show week long lifetime luminescence (phosphorescence) in the 450 - 600 nm region in bound to the kinases form (fluorescence spectrometer LS 55 from Perkin Elmer and luminescence platereader PHERAStar fom BMG were used for measurements throughout this work).

The fluorescent acceptor F of the luminescent probe of the current invention should exhibit a large extinction coefficient and a good quantum yield, and its excitation spectrum should overlap with the emission spectrum of the long lifetime luminescence donor P. Fluorescence labels whose excitation spectrum better overlaps with the emission spectrum of the luminescence donor, who have longer emission wavelengths, and greater photostability are preferred, but the choice of the label also depends on the origin of the analytical instrument used for the assay and special analytical situation. Fluorescent dyes that may be useful in the present invention include inter alia fluorescein and fluorescein derivatives, rhodamine and rhodamine derivatives, cyanine dyes and many other fluorescent labels that are produced and sold by different companies (e.g., Invitrogen, PromoCell, ATTO-TEC GmbH, GE Heathcare, Sigma-Aldrich). Other fluorescent labels that absorb light in the 400-900 nm range and emit light in the 450-1000 nm range are suitable for the application as the fluorescent acceptor moiety F of the luminescence probes of the present invention. Well-known fluorescence dyes FITC, TAMRA Cy3, Cy3B, Cy5, Bodipy 564/570, Texas Red, Alexa Fluor 647, PromoFluor 647 and others have shown suitable optical characteristics when used in combination with thiophene and selenophene-based fragments P. Fluorescent probes incorporating mineral fluorescent labels, e.g., quantum dots, may be useful for special applications. Luminescent probes P-L-F (e.g., compounds ARC-669, ARC-676, ARC-679, ARC-1063, ARC-1122, ARC-1128, ARC-1130, ARC-1133, ARC-1136, ARC-1139, and ARC-1144 in Table 1) are excited at wavelengths below 400 nm with single-photon excitation and at wavelengths over 600 nm when multiphoton excitation is used. The emitted radiation is measured at wavelengths 450 - 1000 nm.

5-carboxytetramethylrhodamine (5-TAMRA) is a good fluorescent label to be used as a fluorescence acceptor of a luminescence probe of the current invention, as the dye is a single-position isomer, the dye is exited at a suitable wavelength, it has high brightness and great photostability.

Many other fluorescent labels are suitable for the application as the acceptor moiety F of the luminescence probes. These labels are described in the Handbook: [The Handbook - A Guide to Fluorescent Probes and Labeling Technologies Web Edition of The Handbook, Tenth Edition 2006 Invitrogen Corporation].

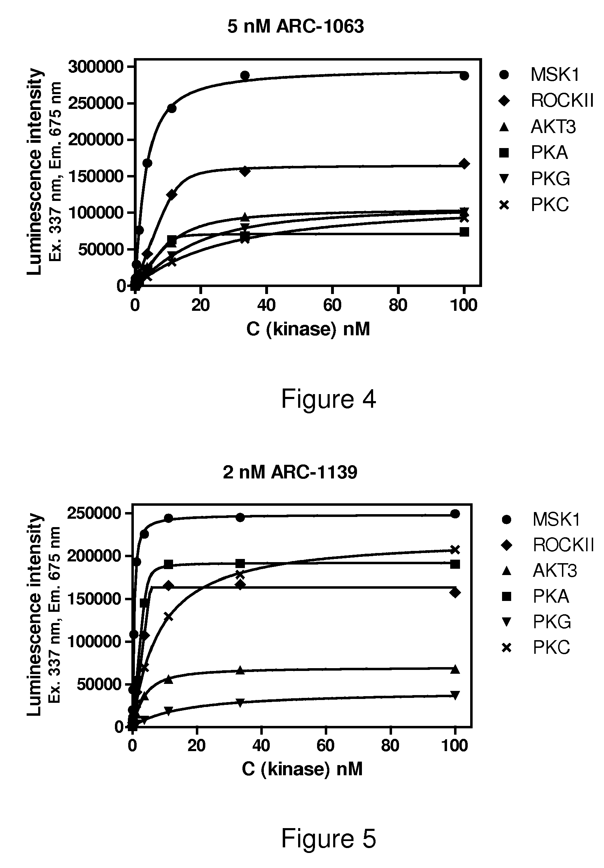

In the case of other luminescent probes the linker unit L may be a C₁-C₂₀ alkylene group, a C₁-C₂₀ alkenylene group, a C₁-C₂₀ alkynylene group, wherein one or more of the CH₂ groups present in the C₁-C₂₀ alkylene group, C₁-C₂₀ alkenylene group, C₁-C₂₀ alkynylene group is optionally replaced with -O-, -C(O)-, -C(O)N-, -S-, - S(O)-, -SO₂-, -N(R)-; R is H or C₁₋₆ alkyl. The novel fluorescently labelled thiophene- or selenophene-based luminescent probes possess 15 - 500 µs lifetimes when these probes are bound to the purine-binding site of a PK (FIG. 1). Binding of the probes to different kinases leads to a luminescence signal whose magnitude is dependent on the origin of the probe and that of the kinase (FIG. 5). When longer delay times (e.g., > 50 µs) after the excitation pulse are used for the measurement of the emission signal, the binding of the probes to the target PKs leads to 50 - 2500-fold increase of the luminescence signal. Microsecond lifetimes of the probes enable the performance of time gated measurements with the application of commercial platereaders and microscopes.

Another embodiment of the present invention is the use a PK inhibitor possessing a long lifetime luminescence donor properties (P-L) but lacking a fluorescent dye, e.g., ARC-668 (Table 1). Disclosed herein is a PK inhibitor (P-L) in combination with a PK that is labeled with an organic dye, fused with a fluorescence protein or bound to specific fluorescently labelled antibody.

Thus an aspect of the invention is use of a photoluminescent probe that has structure P-L wherein P contains a fragment that has luminescence donor properties and that specifically associates with the purine-binding pocket of a target protein kinase and L is a linker system; said probe being selected from ARC-668, ARC-1121, ARC-1123, ARC-1129, ARC-1132, ARC-1135 and ARC-1138; wherein the target protein kinase is a protein kinase labelled with a fluorescent dye F;
wherein the excitation spectrum of the fluorescent dye F overlaps with the emission spectrum of P;
in an in vitro method to monitor kinase activity in living cells;
wherein the luminescence of a photoluminescent probe is measured in time-delayed mode;
wherein said probe, when bound to the purine-binding pocket of the target protein kinase, is excited with a single photon excitation pulse in the wavelength range 280-450 nm and the probe emits luminescence radiation in the wavelength range 450-1000 nm;
the time-delay being such that the luminescence radiation emitted by the probe is measured free from 1 to 10 nanosecond-lifetime background fluorescence caused by organic compounds and light scattering.

Other tagging methods (e.g., FlAsH, HaloTag, etc.) can be use for labelling of the target proteins. The binding of the probe P-L to the labelled kinase that leads to intermolecular resonance energy transfer from the bound luminescence donor to the labelled protein (FIG. 2) that can be detected measuring the signal emitted by the fluorescence acceptor.

A special embodiment of the invention is the luminescent probe ARC-669 with the structure:

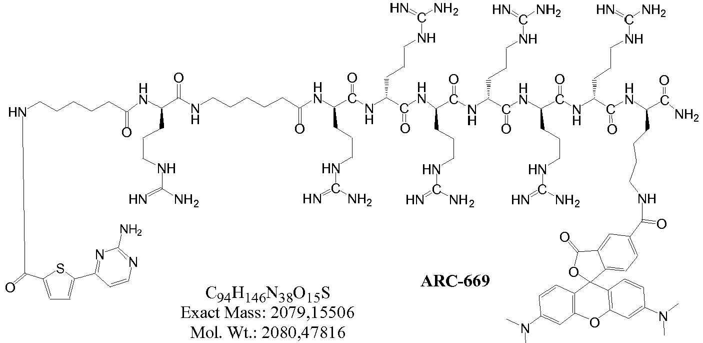

The probe, ARC-669, has high affinity (K_{d}= 0.1 nM) towards the catalytic subunit of cAMP-dependent protein kinase (PKAc). It was previously described as a fluorescent probe for fluorescence polarization/anisotropy and fluorescence intensity measurements in steady state conditions [Lavogina et al., Biochim Biophys Acta. 1804 (2010) 1857]. It is displaced from its complex with the kinase by ATP-competitive inhibitor H89, protein substrate competitive inhibitor protein PKI, and bisubstrate inhibitor AdcAhx(D-Arg)₆-NH₂ (ARC-902). Now the compound ARC-669 was developed into a long life-time luminescence probe that binds to several PKs with high affinity (Table 2). The probe can be used as a high-affinity luminescent probe for time-resolved luminescence assays.

When the solution of ARC-669 was illuminated at 337 (50) nm its binding to a PK induced 50 - 2500-fold increase of the luminescence signal when 50 µs delay after the excitation pulse and 100 µs measuring window was used for the detection by the intensity of the signal at 590 (40) nm. The luminescence lifetime of the signal was dependent on the origin of the binding PK and it was in the range of 50-300 µs.

According to the general structure P-L-F, in the case of the probe ARC-669 P is represented by a aminopyrimidine moiety and a thiophene residue, linker L incorporates a carboxylate group, a 6-aminohexanoic acid residue, a D-arginine residue, a second 6-aminohxanoic acid residue, six D-arginine residues and a D-lysine residue with amidated carboxylate group, F is a 5-carboxytetramethylrhodamine dye connected to the side chain of the lysine residue.

The potential of ARC-669 to inhibit different PKs was tested in a panel of 115 kinases. It was established that ARC-669 binds with high affinity to many PKs (Table 2) and thus it can be used as a luminescent probe with several PKs.

Authors of the present invention have recently shown that highly potent ATP-competitive conjugates of adenosine analogs and arginine-rich peptides are cell membrane permeable [Uri et al., Bioorg. Med. Chem. Lett. 12 (2002) 2117; Enkvist et al., Bioorg. Med. Chem. Lett. 19 (2009) 6098; Vaasa et al., Biochem Biophys Res Commun. 397 (2010) 750], therefore some of the fluorescent probes of the present invention are applicable in experiments with live cells, tissues and organisms.

TR-FRET assays are homogeneous and as such they do not require separation steps like chromatography, filtration, centrifugation, precipitation or electrophoresis. Due to the high sensitivity of TR luminescence assay it is well suited for the assay miniaturization, and the assays can be performed with equal success in a cuvette of a fluorescence spectrometer and in low □l volumes in wells of 384-well and 1536-well microtiter plates with a fluorescence plate reader with delayed fluorescence measurement possibilities.

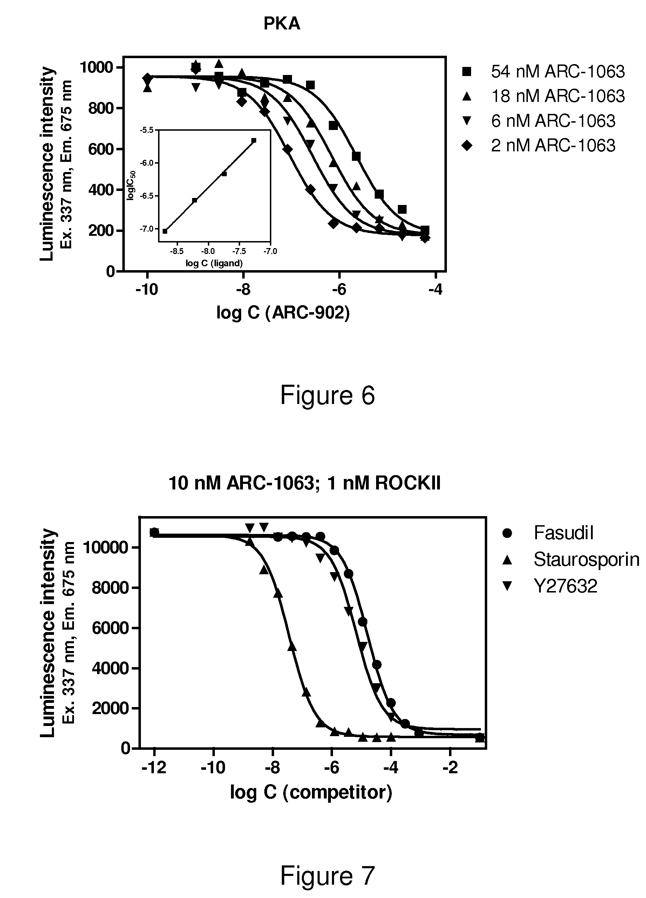

The present invention embodies an assay in which the binding characteristics of the test compounds are determined on the basis of their ability to displace the fluorescent probe from its complex with the kinase. This is described in Example 3 and shown on FIG. 6-8.

The high affinity of the fluorescent probes of the present invention (K_{d} < 1.0 nM) makes it possible to characterize the high-affinity inhibitors with sub-nanomolar and nanomolar affinity. K_{d}-values of the inhibitors could be calculated according to the Cheng-Prusoff equation.

The application of the luminescent probes for the determination of the concentration of the active form of the kinase is another embodiment of the current invention. Determination of the concentration of the special form of a kinase is of paramount importance for many enzyme applications. In kinetic assays the kinase amount in the assay volume is characterized on the activity basis and the molar concentration of the kinase is often not known. Majority of the methods in use for determination of enzyme concentration (Bradford, Lowry, SDS electrophoresis) give the total concentration of the protein (not the concentration of its active form), are inaccurate and need unacceptably large amounts of the protein for analysis.

The application of the photo-luminescent probe of the current invention enables a simple procedure for the determination of the concentration of the active (binding to the fluorescent probe) form of the kinase in the sample (Example 2).

It was established that there was a good proportionality between the concentration of the binding protein (as established with the photo-luminescent probe) and the phosphorylation activity of the solution of the PK.

The luminescent probes of the current invention can be used for measurements with several PKs. Titration of probes ARC-1063 and ARC-1139 with many kinases (PKA, MSK1, ROCKII, AKT3, PKG, PKC) resulted in substantial increase of luminescence emission (FIG. 4 and 5).

A selectivity testing performed (Example 7) for the probe ARC-669 established the inhibitory potency of the compound towards PKs of different groups. Profiling with the kinase panel (Example 7) reveals strong tendency of the compound to inhibit basophilic PKs that points to the active participation of both functionary moieties in the formation of the binary complex with the kinase. In addition to the majority of AGC kinases, many kinases of CAMK and STE groups and some kinases not falling into major groups (classification by [Manning et al., Science 298 (2002) 1912]) were strongly inhibited by the probe. All the inhibited kinases are targets of fluorescent probes of the present invention and the probes can be used in analytical methods concerning these kinases. Probes targeted to PKs of other groups can be developed using luminescence donors and acceptors described in the present invention by adding structural elements that support binding of the probes to these kinases. Additionally, the potential targets of the fluorescent probes of the present invention are different mutated forms of PKs, truncated forms of kinases inactive states of PKs, and pseudokinases.

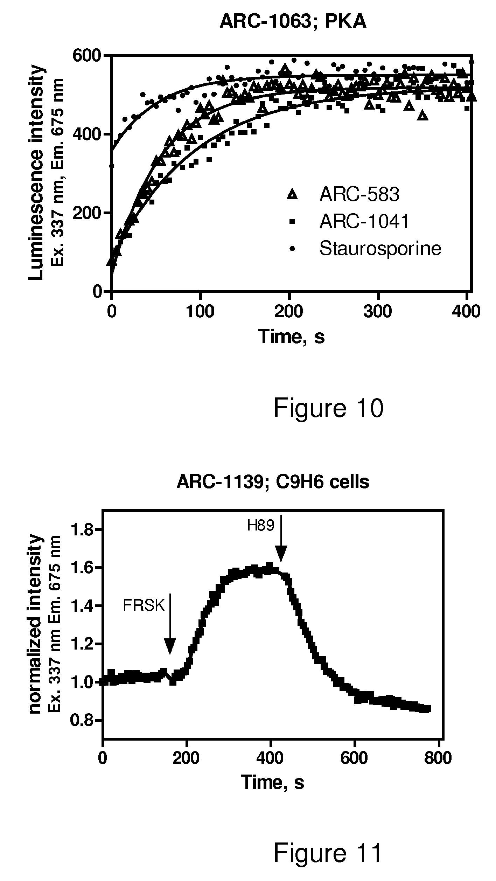

In addition to their use in biochemical assays with purified PKs and PKs in complicated biological solutions (cell lysates, tissue extracts, body fluids) the probes of the present invention can be used for monitoring the activity of PKs in living cells (FIG. 11 - 13). This enables the application of the probes for monitoring the physiological response of the cells to activation of membrane receptors. Both agonists and antagonists of receptors can be screened and characterized with the application of the probes as far as these receptors activate intracellular signalling cascades involving PKs. Regulation of PKA, e.g., mediated via Gₛ and Gᵢ proteins (FIG. 11 - 13), but also isoforms of PKC and Rho-kinase can be monitored with luminescent probes of the present invention in living cells and cell lysates. Thus these probes can be used for screening and characterization of ligands leads to intracellular activation PKs.

PKs are acknowledged disease biomarkers and changes in the level of their activity in body fluids and tissues signal about diseases like cancer, diabetes, atherosclerosis, etc. The luminescent probes of the current invention can be used for diagnosis of these diseases, for monitoring of the efficiency of the cure of patients, and for supporting drug discovery and development in pharmaceutical industry. Cancer cells leak out PKAc and the higher level of its activity in blood plasma and serum of cancer patients has been reported. [WO2000/079281] The luminescent probes of the present invention can be used for high throughput analysis of plasma samples for establishment of the level of PKAc activity (FIG. 14 and Example 9). The assay based on the use of luminescent probes of the current invention has very high sensitivity and affords the determination of the concentration of PKA with very low limit of quantification (LoQ = 15 pM, 20 □L volume in a well of 384-well microtiter plate). The use of plasma samples makes the diagnosis of cancer minimally invasive for patients and low price and high throughput format of analysis of the samples support the development of analysis methods based on luminescent probes of the present invention for massive analysis of blood samples at hospital laboratories and blood banks for early diagnosis of cancer.

### EXPERIMENTAL PART

### Abbreviations and definitions

Adc - 5'-adenosine carboxylic acid or 1-(6-amino-9H-purin-9-yl)-1-deoxy-β-D-ribofuranuronic acid (CAS 3415-09-6);
Ahx - 6-aminohexanoic acid;
Arg - arginine;
ARC - adenosine-arginine conjugate;
ATP - adenosine-5'-triphosphate;
Boc - tert-butoxycarbonyl;
cAMP - cyclic adenosine 3',5'-monophosphate;
DIPEA - N,N-diisopropylethylamine;
DMF - dimethylformamide;
DMSO - dimethyl sulfoxide;
DTT - dithiothreitol
Fmoc - 9-fluorenylmethoxycarbonyl;
HOBt - 1-hydroxybenzotriazole;
HPLC - high performance liquid chromatography;
Ip: 2',3'-O-isopropylidene;
MALDI TOF MS, matrix assisted laser desorption ionisation time-of-flight mass spectrometry;
NMR - nuclear magnetic resonance;
Pbf - 2,2,4,6,7-pentamethyldihydrobenzofuran-5-sulfonyl;
PK - protein kinase;
PKA - cAMP-dependent protein kinase;
PKAc - catalytic subunit of PKA
RP - reverse phase;
TFA - trifluoroacetic acid;
TLC - thin layer chromatography.
**ARCs** - conjugates of adenosine analogues and arginine-rich peptides are bisubstrate inhibitors of basophilic protein kinases

**Bisubstrate inhibitors** simultanously associate with two substrate-binding sites of PK and thus combine two inhibitory domains. [Lavogina et al., ChemMedChem 5 (2010) 23] The principle advantage of bisubstrate inhibitors is their ability to generate more interactions with the target enzyme that could result in improved affinity and selectivity of the conjugates, when compared with single-site inhibitors.

**Oligopeptide** refers a short polymer of amino acids (2-20 amino acids) which are joined together by peptide bonds.

*The following terms are defined according to the* book Lakowicz Principles of fluorescence spectroscopy, 3rd edition, 2006*.*

**FRET** - Forster-type non-radiative dipole-dipole energy transfer [Förster, Ann. Physik. 6 (1948) 55] takes place between two molecules in condition where their energies (emission of donor D with absorption of acceptor A) overlap and they are located at a suitable distance from each other. Typically, a FRET occurs at distance less than 100 A between the donor and acceptor.

**TRF** - time-resolved fluorescence. Fluorescence measurements can be broadly classified into two types of measurements: steady-state and time-resolved (TR). Steady-state measurements, the most common type, are those performed with constant illumination and observation. TR measurements are performed after short-pulse excitation of the fluorophore and the emission signal is detected after delay for a certain time period (measurement window). Although measurement of the time-resolved emission requires special instruments, such technology is widely used in biological research because of the increased information available from the data as compared with stationary or steady-state measurements. **Photoluminescence** is the emission of light from a substance as a result of absorption of electromagnetic radiation, the emitted light usually has a longer wavelength than the incident radiation. Photoluminescence is formally divided into two categories-fluorescence and phosphorescence-depending on the nature of the excited state. In excited singlet states, the electron in the excited orbital is paired (by opposite spin) to the second electron in the ground-state orbital. Consequently, return to the ground state is spin allowed and occurs rapidly by emission of a photon. The emission rates of fluorescence are typically 10⁸s⁻¹, so that a typical fluorescence lifetime is in the range of 1-10 ns.

**Phosphorescence** is emission of light from a triplet excited states, in which the electron in the excited orbital has the same spin orientation as the ground-state electron. Transitions to the ground state are forbidden and the emission rates are slow (10⁻³ to 100 s⁻¹), so that phosphorescence lifetimes are typically milliseconds to seconds.

The luminescence **lifetime** refers the average amount of time a fluorophore remains in the excited state (following excitation) prior to return to the ground state. Typically, fluorescence lifetimes are near 1 - 10 ns and a phosphorescence lifetimes are in the range of microseconds to seconds.

### EXAMPLES

### EXAMPLE 1. SYNTHETIC PROCEDURES

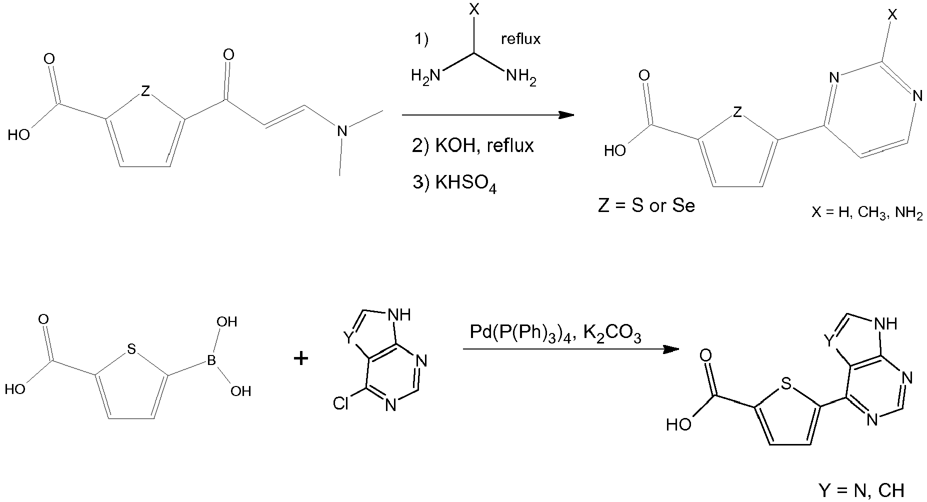

### Synthesis of adenosine mimetics

### 5-(3-Dimethylaminoacryloyl)thiophene-2-carboxylic acid methyl ester

2 ml (15 mmol) of N,N-dimethylformamide dimethyl acetal was added to the suspension of 5-acetyl-2-thiophenecarboxylic acid (1013 mg, 5.95 mmol) in DMF (5 ml) and the reaction mixture was refluxed overnight. 100 ml of hexane/tBuOMe (1:1) was added to the cooled reaction mixture and the precipitate was separated by filtration to yield 1140 mg (80%) of the product.

### 5-(2-aminopyrimidin-4-yl)thiophene-2-carboxylic acid

1 (466 mg, 1.95 mmol), guanidine hydrochloride (468 mg, 4.9 mmol) and K₂CO₃ (888 mg, 6.4 mmol) were refluxed in DMF (15 ml) for 3 days. The solvents were removed and the residue was suspended in solution of KOH (0.5 M, 20 ml) and refluxed overnight until clear solution was formed. The product was precipitated with 20% solution of KHSO₄, filtrated, washed and tried to yield the product (360 mg, 83.5 %). TLC (DCM/MeOH/AcOH, 9:1:1) R_{f} = 0.47.

¹H NMR (CD₃SOCD₃) δ 6.85 (2H, br, NH₂), 7.17 (1 H, d, *J* = 5.2 Hz), 7.76 (1 H, d, *J* = 4 Hz), 7.92 (1 H, d, J = 4 Hz), 8.34 (1 H, d, J = 5.2 Hz), 13.33 (1 H, br, COOH). ¹³C NMR (CD₃SOCD₃) δ 104.6, 127.7, 133.7, 136.6, 148.6, 158.0, 159.3, 162.7, 163.5.

### 5-(2-methylpyrimidin-4-yl)thiophene-2-carboxylic acid

The compound was synthesized as 5-(2-aminopyrimidin-4-yl)thiophene-2-carboxylic acid except acetamidine was used instead of guanidine. Yield 85 %. ¹H NMR (CD₃SOCD₃) δ 2.63 (3H, s, CH₃), 7.78 (1 H, d, *J* = 4.0 Hz), 7.89 (1 H, d, *J* = 5.4 Hz), 8.07 (1 H, d, *J* = 4.0 Hz), 8.76 (1 H, d, *J* = 5.4 Hz), 14.40 (1 H, br, COOH). ¹³C NMR (CD₃SOCD₃) δ 25.3, 112.7, 128.2, 133.5, 137.6, 147.2, 157.2, 158.0, 162.2, 167.5.

### 5-(pyrimidin-4-yl)thiophene-2-carboxylic acid

The compound was synthesized as 5-(2-aminopyrimidin-4-yl)thiophene-2-carboxylic acid except formamidine was used instead of guanidine. Yield 74 % ¹H NMR (CD₃SOCD₃) δ 7.79 (1 H, d, *J* = 4.0 Hz), 8.05 - 8.15 (2H, m), 8.88 (1 H, d, *J* = 5.4 Hz), 9.17 (1 H, d, *J* = 1.2 Hz).

¹³C NMR (CD₃SOCD₃) δ 115.7, 128.5, 133.5, 138.1, 146.5, 157.1, 157.8, 158.5, 162.2.

### 5-(2-aminopyrimidin-4-yl)selenophene-2-carboxylic acid

Selenophene-2-carboxylic acid was refluxed overnight in acetic anhydride in the following synthesis was carried out as described for 5-(2-aminopyrimidin-4-yl)thiophene-2-carboxylic acid (except 5-(3-dimethylaminoacryloyl)selenophene-2-carboxylic acid methyl ester did not precipitate from ether/alkane mixture and the solvents were removed before next step). The yield of 5-(2-aminopyrimidin-4-yl)selenophene-2-carboxylic acid from selenophene-2-carboxylic acid was 17%. ¹H NMR (CD₃SOCD₃) δ 6.80 (2H, br, NH₂), 7.16 (1 H, d, *J* = 5.1 Hz), 7.97 (1 H, d, *J* = 4.2 Hz), 8.12 (1 H, d, *J* = 4.2 Hz), 8.32 (1 H, d, *J* = 5.1 Hz).

¹³C NMR (CD₃SOCD₃) δ 103.7, 129.6, 136.3, 142.2, 156.0, 159.2, 159.4, 163.6, 164.0.

### 5-(9H-purin-6-yl)thiophene-2-carboxylic acid and 5-(7H-pyrrolo[2,3-d]pyrimidin-4-yl)thiophene-2-carboxylic acid

6-Chloropurine (108 mg, 0.70 mmol) or 6-chloro-7deazapurine (105 mg, 0.68 mmol), 5-carboxythiophene-2-boronic acid (145 mg, 0.84 mmol), K₂CO₃ (315 mg, 2.3 mmol) and tetrakis(triphenylphosphine)-palladium (120 mg, 0.1 mmol) were stirred overnight at 100 °C under argon. The solvents were removed under vacuum. The residues were dispersed in water and filtrated. The filtrates were acidified with KHSO₄ and the precipitates were collected. The products were used in the solid phase synthesis without further purification.

### Solid-phase synthesis of peptide conjugates

Peptide fragments were prepared by using traditional Fmoc solid-phase peptide synthesis methods on Rink amide MBHA resin. Protected amino acids (3 equivalents) were dissolved in DMF and activated with HBTU/HOBt (2.94 eq each) in DMF/N-methylmorpholine. Coupling solutions were added to the resin and shaked 40-60 min. The completeness of each step was monitored with Kaiser-test, which was followed by deprotection of Fmoc-group by 20% piperidine solution in DMF (20 min). Fmoc-protected linkers were attached to the peptide part following the same protocol.

1.5 eq of a thiophene or selenophene containing precursor was activated with HOBt/HBTU (1.47 eq each) in DMF/N-methylmorpholine and added to the resin and shaked at least 3h. Finally the resins were washed 5 times with each solvent (DMF, isopropanol, DCE) and dried. Treatment with TFA/H₂O/triisopropylsilane (90/5/5 by volume) for 2-3 h was used as standard cleavage procedure. The products were purified by reversed-phase HPLC and lyophilized.

### Labelling of the ARC-s with fluorescent dyes (a typical example of the procedure)

1 □mol of a lysine-containing ARC-type inhibitor and 1.3 □mol *N-*hydroxysuccinimide esters of the fluorescent dye were dissolved in 50 □l of DMSO and 10 □l triethylamine. The solution was kept 3 h at room temperature and then the solvent were removed in high vacuum. The residue was purified by reversed-phase HPLC and lyophilized to give the desired products in 40 - 70% of yields.

### EXAMPLE 2. TITRATION OF LUMINESCENT PROBES WITH PROTEIN KINASES

Biochemical binding experiments were performed in black, low-volume, 384-well nonbonding surface NBS microplates (Corning) on a PHERAstar plate reader (BMG Labtech) with TRF optic modules [ex. 337(50) nm, em. 675(50) and 620(20) nm] or [ex. 337(50) nm, em. 590(50) and 545(10) nm] using the time-resolved fluorescence mode. The microplates were incubated for 15 min at 30 °C before each measurement. The luminescent probe was excited with a short pulse of flashes (200 flashes per datapoint) at 337 nm, followed by 50 µs delay time and subsequent measurement of the luminescence using a constant acquisition window (150 µs) at the wavelength corresponding to the emission maximum of the conjugated fluorescent dye [590(50 )nm or 675(50) nm].

The concentration series of kinases MSK1, ROCKII, AKT3, PKAc, PKG I□, or PKC δ (3-fold dilutions) was made in the assay buffer (150 mM NaCl, 50 mM Hepes pH=7.5, 5 mM DTT, 7.5 µM BSA) and the fixed concentration of the luminescent probe ARC-1063 or ARC-1139 (5 nM or 2nM, respectively) was added to each well, incubated and measured as described above. Structures of luminescent probes ARC-1063 and ARC-1139 are listed in Table 1.

Selenophene-based probes (e.g., ARC-1139) gave generally several-fold higher intensity of the emitted signal than the thiophene-based counterparts (e.g., ARC-1063). Minding to the kinase MSK1 led to the strongest signal with both of the luminescent probes, ARC-1063 and ARC-1139. All the tested PKs of the AGC group produced strong TR luminescence signal when bound to the luminescent probes of the current invention (FIG. 4 and 5).

### EXAMPLE 3. COMPETITIVE DISPLACEMENT OF A LUMINESCENT PROBE BY PK INHIBITORS

Displacement assay was performed with a concentration series of the competitive compound (3-fold dilutions) in assay buffer (150 mM NaCl, 50 mM Hepes pH=7.5, 5 mM DTT, 7.5 µM BSA) and the fixed concentration of luminescent probe ARC-1063 in complex with a PK was added. The displacement curves were fitted to a sigmoidal dose-response model to obtain IC₅₀ values. K_{d} values were calculated using the Cheng-Prusoff equation (K_{d} = IC₅₀/(1 + C_{ARC}-_{Lum}/K_{d} ARC-Lum)).

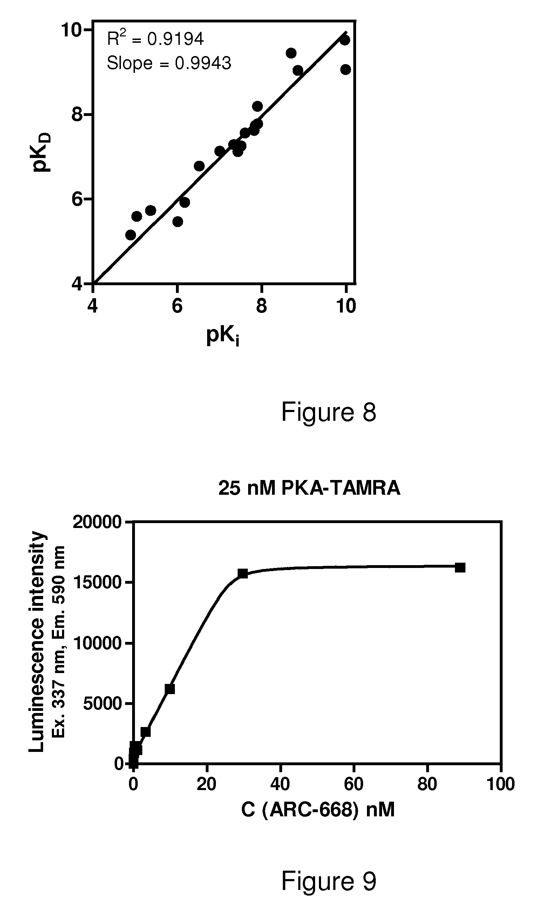

ARC-Lum probes were successfully displaced from kinase complexes by various ATP-competitive, protein substrate competitive and bisubstrate inhibitors (FIG. 6 and 7). IC₅₀-values of competitive inhibitors were dependent on the concentration of ARC-1063 proving the validity of the Cheng-Prusoff equation in this assay (FIG. 6). Calculated K_{d}-values were in good correlation with Kᵢ-values from enzyme kinetic measurements (FIG. 8). Reliable signals for displacement measurements were obtained even at low, subnanomolar concentration of the PKs tested.

### EXAMPLE 4. ENHANCEMENT OF LUMINESCENCE BY INTERMOLECULER FRET (FIG. 2)

A sample of the recombinant kinase PKAc protein was labeled with 5-TAMRA-NHS ester and the labelled protein PKAc-TAMRA was purified by using a Sephadex column. The concentration series of ARC-668 (structure in Table 1) was made in the assay buffer and fixed concentration of PKAc-TAMRA (25 nM) was subsequently added. The luminescence signal was acquired by using the optic module [ex. 337(50) nm, em. 590(50). Intermolecular enhancement of kinase induced long lifetime luminescence by the acceptor was shown to be effective when PKAc was chemically labeled with 5-TAMRA (FIG. 9).

### EXAMPLE 5. MEASUREMENT OF DISSOCIATION KINETICS

Measurements of dissociation kinetics of non-labeled inhibitors from the inhibitor-kinase complexes (residence time) were carried out by mixing the solution of the examined inhibitor (5 - 10 nM) with PKAc (10 nM), and incubation of the solution for 15 min at 27 °C, followed by addition of a high concentration of the luminescent probe ARC-1063 (final concentration of 100 - 300 nM), and measurement of the luminescence signal at 5 s time intervals (FIG. 10). The collected data were fitted to the first-order kinetic model yielding the dissociation rate constants for the examined inhibitors.

Dissociation half-lives of PKAc complexes with the tested inhibitors were the following: ARC-1041, 63 s; ARC-583, 43 s; and staurosporine, 12 s (FIG. 10).

### EXAMPLE 6. MONITORING OF KINASE ACTIVITY IN LIVING CELLS

CHO, C9H6 and HEK cells were cultured in F12 Nutrient Mixture supplemented with 10% fetal bovine serum, 2 mM L-glutamine, 100 U/ml penicillin and 100 µg/ml streptomycin at 37°C in humidified atmosphere containing 5% CO₂. In case of C9H6 cells, 300 µg/ml of Zeocine™ and 800 µg/ml of Geneticin® were additionally introduced to the nutrient mixture. Cells were seeded to 96-well plates (for plate reader experiments) and grown to 60%-80% confluency. The medium was replaced with fresh serum-free medium containing the luminescent probe ARC-1139 (10 µM) and incubated for 1 hour at 37°C. Subsequently, the cells were washed three times with PBS and analyzed on a PheraStar platereader with TRF optic modules [ex. 337(50) nm, em. 675(50) and 620(20) nm] using the time-resolved fluorescence measurement mode. Forskolin (final concentration of 25 µM) and H89 (final concentration of 100 µM) were added to the cells during the measurement (after 3 min and 7 min from the start of data acquisition, respectively)

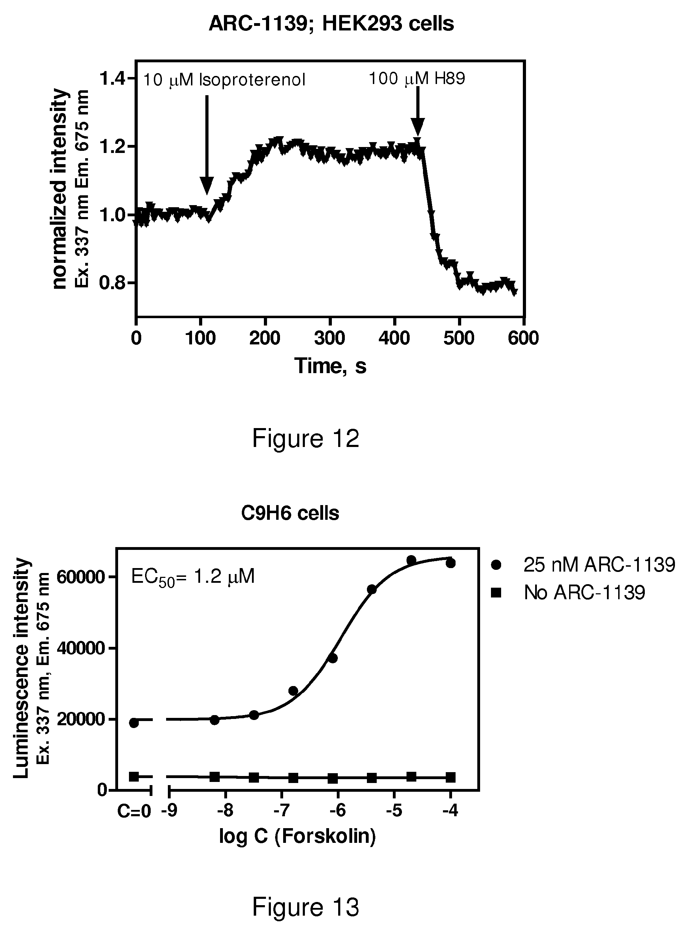

Forskolin caused up to two fold increase of the luminescence from live cells loaded with ARC-1139. The signal decreased after addition of 100 □M H89 that displaces ARC-1139 from its complex with PKAc (FIG. 11). This proves that the luminescent probes can be used for monitoring of kinase activities and effect of the agonists of Gₛ protein-coupled receptors in living cells (FIG. 12).

### EXAMPLE 7. PK SELECTIVITY PROFILING OF A LUMINESCENT PROBE

Selectivity testing (Table 2) was performed on the commercial basis at the Division of Signal Transduction Therapy, University of Dundee. ATP concentrations that were used in testing were close to the Kₘ value of the kinase.

**Table. 2. Percentages of residual activities of the kinase reaction in the presence of 1 □M ARC-669**

| | | | | | | | |
|---|---|---|---|---|---|---|---|
| MKK1 | 69 | | CHK1 | 29 | | PAK6 | 35 |
| MKK2 | 68 | | CHK2 | 28 | | MST2 | 94 |
| MKK6 | 107 | | GSK3b | 77 | | MST4 | 45 |
| ERK1 | 114 | | CDK2-Cyclin A | 78 | | GCK | 10 |
| ERK2 | 80 | | PLK1 | 101 | | MINK1 | 51 |
| JNK1 | 91 | | Aurora A | 86 | | MEKK1 | 19 |
| JNK2 | 85 | | Aurora B | 81 | | MLK1 | 60 |
| JNK3 | 77 | | LKB1 | 101 | | MLK3 | 70 |
| p38a | | | | | | | |
| MAPK | 55 | | AMPK | 36 | | TAO1 | 94 |
| p38b MAPK | 71 | | MARK1 | 65 | | ASK1 | 74 |
| p38g MAPK | 104 | | MARK2 | 77 | | TAK1 | 13 |
| p38d MAPK | 104 | | MARK3 | 20 | | IRAK4 | 82 |
| ERK8 | 23 | | MARK4 | 86 | | RIPK2 | 47 |
| RSK1 | 2 | | BRSK1 | 44 | | TTK | 66 |
| RSK2 | 4 | | BRSK2 | 77 | | MPSK1 | 97 |
| PDK1 | 88 | | MELK | 31 | | Src | 106 |
| PKBa | 1 | | NUAK1 | 12 | | Lck | 11 |
| PKBb | 4 | | CK1 | 72 | | CSK | 51 |
| SGK1 | 8 | | CK2 | 7 | | YES1 | 64 |
| S6K1 | 4 | | DYRK1A | 27 | | ABL | 88 |
| PKA | 1 | | DYRK2 | 65 | | BTK | 36 |
| ROCK 2 | 10 | | DYRK3 | 18 | | JAK2 | 80 |
| PRK2 | 4 | | NEK2a | 55 | | SYK | 73 |
| PKCa | 15 | | NEK6 | 71 | | ZAP70 | 82 |
| PKCγ | 13 | | IKKb | 50 | | TIE2 | 86 |
| PKCz | 10 | | IKKe | 37 | | BRK | 100 |
| PKD1 | 37 | | TBK1 | 27 | | EPH-A2 | 98 |
| STK33 | 76 | | PIM1 | 3 | | EPH-A4 | 76 |
| MSK1 | 3 | | PIM2 | 15 | | EPH-B1 | 90 |
| MNK1 | 74 | | PIM3 | 7 | | EPH-B2 | 96 |
| MNK2 | 76 | | SRPK1 | 66 | | EPH-B3 | 75 |
| MAPKAP-K2 | 13 | | EF2K | 43 | | EPH-B4 | 98 |
| MAPKAP-K3 | 31 | | HIPK1 | 94 | | FGF-R1 | 54 |
| PRAK | 63 | | HIPK2 | 63 | | HER4 | 82 |
| CAMKKb | 41 | | HIPK3 | 97 | | IGF-1R | 38 |
| CAMK1 | 3 | | CLK2 | 1 | | IR | 52 |
| SmMLCK | 46 | | PAK2 | 43 | | IRR | 32 |
| PHK | 32 | | PAK4 | 32 | | TrkA | 101 |
| DAPK1 | 20 | | PAK5 | 18 | | VEG-FR | 25 |

Data of selectivity profiling indicates that the luminescent probe ARC-669 has adequate affinity towards most of the AGC kinases and several PKs from other groups to be used in binding experiments with detection of kinase-induced long lifetime luminescence. Thus the probe can be used for high throughput characterization of inhibitors of many PKs.

### EXAMPLE 8. MEASUREMENT OF CELLULAR ACTIVATION OF PKA VIA LYSATE ANALYSIS

CHO cells stably over-expressing PKAc were grown to 80% confluence on black 96-well micro plates (Corning). Cells were washed once with 300 µL HBSS (Gibco). Thereafter 25 µL of 100 µM IBMX in HBSS containing different concentrations of Forskolin (0 - 100 µM) were added to the cells and incubated for 10 minutes at 30°C. The cells were lysed by adding 25 µL of cell lysis buffer (Invitrogen NP40 cell lysis buffer) containing additionally 1% Triton X, 1x Protease inhibitor cocktail (Sigma-Aldrich Protease inhibitor cocktail for general use), 10 µM PMSF and 0 or 50 nM ARC-1139. The cells were lysed at 30 °C for 10 minutes and the luminescence intensities were measured using a PheraStar plate reader (BMG) with HTRF module TRF excitation filter [(BMG 337), emission at 675(50) nm; 200 flashes, delay 50 µs, integration time 150 µs]. The results were fitted to sigmoidal dose-response model using GraphPad Prism 5 software.

The obtained luminescence intensities were significantly higher when 50 nM ARC-1139 (final concentration in the cell lysate 25 nM) was included in the lysis buffer. The increase of the luminescence signal arises from the formation of the complexes of ARC-1139 and different kinases present in the cell lysate. The luminescence intensity is significantly increased (FIG. 13) as the concentration of Forskolin a cell permeable activator of adenylate cyclase is increased. Adenylate cyclase catalyses the formation of cAMP from ATP and cAMP in turn activates PKA. Thus, luminescent probes of the current invention are cAMP sensors and as such can be used for monitoring the activity of receptor systems. They can be used for screening agonists and antagonists of metabotropic receptors related via G-proteins (e.g., Gₛ and Gᵢ) to the intracellular activation of adenylate cyclises

### EXAMPLE 9. MEASUREMENTS OF KINASE ACTIVITY IN BLOOD SERUM

To human plasma from the local blood bank a concentration series of recombinant PKA was added to the plasma. 20 µL of each plasma sample was added to 384-well micro plate (Corning) already containing 5 µL of 10 nM ARC-1063 (final concentration 2 nM) or 5 µL of 10 nM ARC-1063, 1 µM PKI, 1 mM ATP and 100 mM MgAcetate (final concentrations 5 nM, 200 nM, 200 µM and 20 mM respectively). The samples were incubated at 30°C for 15 minutes and the luminescence intensities were measured using a PheraStar plate reader (BMG) with HTRF module [TRF excitation filter (BMG 337), emission at 675(50) nm; 200 flashes, delay 100 µs, integration time 100 µs]. All samples were analysed as three replicate points and plotted against the concentration of PKA added to each samples (FIG. 14). The results were fitted to linear regression using GraphPad Prism 5 software.

The obtained luminescence signal is directly proportional to the amount of recombinant PKAc added to the plasma (FIG. 14). Moreover, if PKI, a specific competitive inhibitor of PKAc, is added to the binding assay in the presence of ATP and Mg²⁺ ions the complex of ARC-1063 and PKAc is not formed and only background luminescence (originating from blood components) is detected. Therefore, from the difference of the luminescence signal obtained from the plasma samples in the absence and presence of PKI, the concentration of the endogenous PKAc in plasma can be determined. This sensitive assay has very low limit of quantification (LoQ = 15 pM, 20 µL volume in a well of 384-well microtiter plate) that enables the monitoring of PKAc activity in blood samples.

## Claims

1. A binding assay method for protein kinases wherein the luminescence of a photoluminescent probe bound to a protein kinase is measured, wherein the probe has structure P-L-F wherein P contains a fragment that has luminescence donor properties and that specifically associates with the purine-binding pocket of the target protein kinase, L is a linker system and F is a fluorescence acceptor dye, said probe containing a heteroaromatic fragment based on a thiophene, selenophene or tellurophene moiety linked to an aromatic system Ar and connected via a linker system L with a fluorescent dye F, said probe having structure as follows: 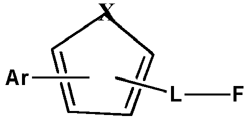
wherein X represents S, Se or Te;
wherein Ar is an aromatic system selected from the list consisting of 2-aminopyrimidine, 2-methylpyrimidine, pyrimidine, pyridine, purine and 7H-pyrrolo[2,3-d]pyrimidine;
wherein the 5-membered S, Se or Te membered ring may additionally optionally incorporate one nitrogen atom;
wherein the 5-membered S, Se or Te containing ring may be optionally substituted by halogeno groups;
wherein the excitation spectrum of the fluorescence acceptor dye F overlaps with the emission spectrum of the luminescent donor P;
comprising (a) exciting said probe bound to the purine-binding pocket of the target protein kinase with a single photon excitation pulse in the wavelength range 280-450 nm and (b) measuring in time-delayed mode luminescence radiation emitted by the probe in the wavelength range 450-1000 nm;
the time-delay being such that the luminescence radiation emitted by the probe is measured free from 1 to 10 nanosecond-lifetime background fluorescence caused by organic compounds and light scattering.

2. An in vitro binding assay method for protein kinases in living cells wherein the luminescence of a photoluminescent probe is detected by using a fluorescence microscope, a fluorescence spectrometer or a fluorescence platereader, wherein the probe has structure P-L-F wherein P contains a fragment that has luminescence donor properties and that specifically associates with the purine-binding pocket of the target protein kinase, L is a linker system and F is a fluorescence acceptor dye, said probe containing a heteroaromatic fragment based on a thiophene, selenophene or tellurophene moiety linked to an aromatic system Ar and connected via a linker system L with a fluorescent dye F, said probe having structure as follows: 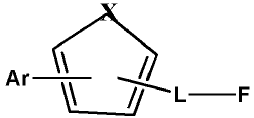
wherein X represents S, Se or Te;
wherein Ar is an aromatic system selected from the list consisting of 2-aminopyrimidine, 2-methylpyrimidine, pyrimidine, pyridine, purine and 7H-pyrrolo[2,3-d]pyrimidine;
wherein L represents 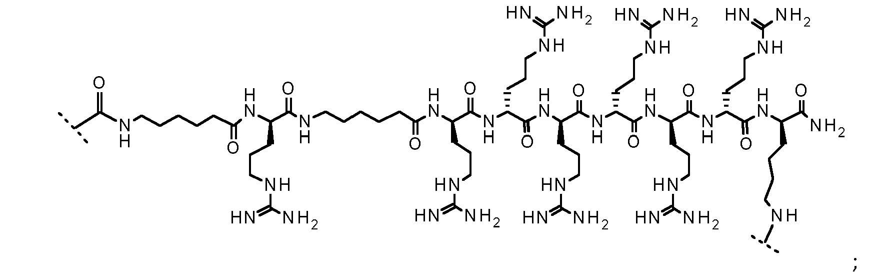
wherein the 5-membered S, Se or Te membered ring may additionally optionally incorporate one nitrogen atom;
wherein the 5-membered S, Se or Te containing ring may be optionally substituted by halogeno groups;
wherein the excitation spectrum of the fluorescence acceptor dye F overlaps with the emission spectrum of the luminescent donor P;
comprising (a) exciting said probe bound to the purine-binding pocket of the target protein kinase with a single photon excitation pulse in the wavelength range 280-450 nm and (b) time-delayed detection of luminescence radiation emitted by the probe in the wavelength range 450-1000 nm;
the time-delay being such that the luminescence radiation emitted by the probe is measured free from 1 to 10 nanosecond-lifetime background fluorescence caused by organic compounds and light scattering.

3. A binding assay method according to claim 1 wherein the fragment P-L of the probe is a bisubstrate inhibitor of the protein kinase.

4. Use of a photoluminescent probe that has structure P-L-F wherein P contains a fragment that has luminescence donor properties and that specifically associates with the purine-binding pocket of a target protein kinase, L is a linker system and F is a fluorescence acceptor dye, said probe containing a heteroaromatic fragment based on a thiophene, selenophene or tellurophene moiety linked to an aromatic system Ar and connected via a linker system L with a fluorescent dye F, said probe having structure as follows: 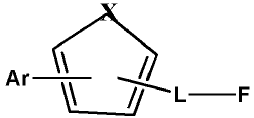
wherein X represents S, Se or Te;
wherein Ar is an aromatic system selected from the list consisting of 2-aminopyrimidine, 2-methylpyrimidine, pyrimidine, pyridine, purine and 7H-pyrrolo[2,3-d]pyrimidine;
wherein the 5-membered S, Se or Te membered ring may additionally optionally incorporate one nitrogen atom;
wherein the 5-membered S, Se or Te containing ring may be optionally substituted by halogeno groups;
wherein the excitation spectrum of the fluorescence acceptor dye F overlaps with the emission spectrum of the luminescent donor P;
in a binding assay for protein kinases;
wherein in the binding assay the luminescence of a photoluminescent probe is measured in time-delayed mode;
wherein said probe, when bound to the purine-binding pocket of the target protein kinase, is excited with a single photon excitation pulse in the wavelength range 280-450 nm and the probe emits luminescence radiation in the wavelength range 450-1000 nm;
the time-delay being such that the luminescence radiation emitted by the probe is measured free from 1 to 10 nanosecond-lifetime background fluorescence caused by organic compounds and light scattering.

5. Use of a binding assay according to claim 1 or claim 3 for quantification of a protein kinase.

6. Use of a binding assay according to claim 1 or claim 3 for determination of the affinity of a protein kinase inhibitor by using the displacement of the probe from a complex of the probe with said protein kinase by a competitive inhibitor.

7. Use of a binding assay according to claim 1 or claim 3 for characterisation of the dissociation kinetics of a complex of an inhibitor with a protein kinase.

8. Use of a binding assay according to claim 1 or claim 3 for quantification of the activity of a protein kinase in a body fluid, cell lysate or tissue extract.

9. Use of a binding assay according to claim 1 or claim 3 for monitoring of receptor activation and cellular concentration of cAMP by analysis of a cell lysate.

10. Use of a binding assay according to claim 1 or claim 3 for quantification of a protein kinase as a biomarker of disease in a body fluid, blood cell or tissue extract.

11. Use of a binding assay according to claim 2 for characterisation of an inhibitor of a protein kinase in living cells.

12. Use of a binding assay according to claim 2 for characterisation of an agonists or antagonists of a Gs and Gi protein-coupled receptor through measurement of the change in luminescence intensity of the probe caused by activation of PKA by the second messenger cAMP in living cells.

13. Use of a binding assay according to claim 2 for characterisation of an agonists or antagonists of a metabotropic or ionotropic receptor through measurement of the change in luminescence of the probe caused by activation of protein kinases other than PKA in living cells.

14. Use of a photoluminescent probe that has structure P-L-F wherein P contains a fragment that has luminescence donor properties and that specifically associates with the purine-binding pocket of a target protein kinase, L is a linker system and F is a fluorescence acceptor dye, said probe containing a heteroaromatic fragment based on a thiophene, selenophene or tellurophene moiety linked to an aromatic system Ar and connected via a linker system L with a fluorescent dye F, said probe having structure as follows: 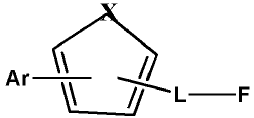
wherein X represents S, Se or Te;
wherein Ar is an aromatic system selected from the list consisting of 2-aminopyrimidine, 2-methylpyrimidine, pyrimidine, pyridine, purine and 7H-pyrrolo[2,3-d]pyrimidine;
wherein the 5-membered S, Se or Te membered ring may additionally optionally incorporate one nitrogen atom;
wherein the 5-membered S, Se or Te containing ring may be optionally substituted by halogeno groups;
wherein the excitation spectrum of the fluorescence acceptor dye F overlaps with the emission spectrum of the luminescent donor P;
wherein the target protein kinase is PKAc;
in a high throughput analysis method for establishment of the level of PKAc activity in a blood plasma sample;
wherein in the method the luminescence of a photoluminescent probe is measured in time-delayed mode;
wherein said probe, when bound to the purine-binding pocket of the target protein kinase, is excited with a single photon excitation pulse in the wavelength range 280-450 nm and the probe emits luminescence radiation in the wavelength range 450-1000 nm;
the time-delay being such that the luminescence radiation emitted by the probe is measured free from 1 to 10 nanosecond-lifetime background fluorescence caused by organic compounds and light scattering.

15. A method or use according to any one of claims 1 to 14 wherein the 5-membered S, Se or Te membered ring is thiophene, selenophene or tellurophene.

16. A method or use according to any one of claims 1 to 14 wherein the probe is selected from ARC-669, ARC-676, ARC-679, ARC-1063, ARC-1122, ARC-1128, ARC-1130, ARC-1133, ARC-1136, ARC-1139 and ARC-1144.

17. Use of a photoluminescent probe that has structure P-L wherein P contains a fragment that has luminescence donor properties and that specifically associates with the purine-binding pocket of a target protein kinase and L is a linker system; said probe being selected from ARC-668, ARC-1121, ARC-1123, ARC-1129, ARC-1132, ARC-1135 and ARC-1138; wherein the target protein kinase is a protein kinase labelled with a fluorescent dye F;
wherein the excitation spectrum of the fluorescent dye F overlaps with the emission spectrum of P;
in an in vitro method to monitor kinase activity in living cells;
wherein the luminescence of a photoluminescent probe is measured in time-delayed mode;
wherein said probe, when bound to the purine-binding pocket of the target protein kinase, is excited with a single photon excitation pulse in the wavelength range 280-450 nm and the probe emits luminescence radiation in the wavelength range 450-1000 nm;
the time-delay being such that the luminescence radiation emitted by the probe is measured free from 1 to 10 nanosecond-lifetime background fluorescence caused by organic compounds and light scattering.

18. A method or use according to any one of claims 1, 3 to 10, 14 or 15 wherein L represents:

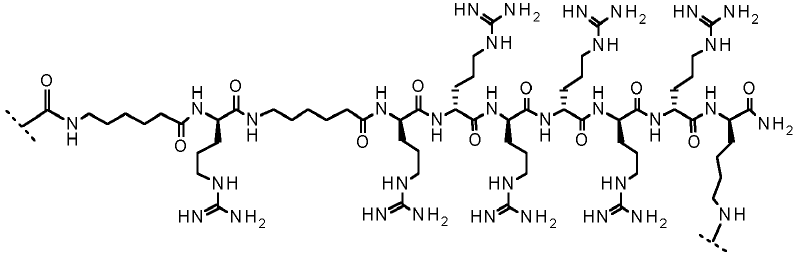

19. A photoluminescent probe having structure P-L -F wherein P contains a fragment that has luminescence donor properties and that specifically associates with the purine-binding pocket of the target protein kinase, L is a linker system and F is a fluorescence acceptor dye, said probe containing a heteroaromatic fragment based on a selenophene or tellurophene moiety linked to an aromatic system Ar and connected via a linker system L with a fluorescent dye F, said probe having structure as follows: 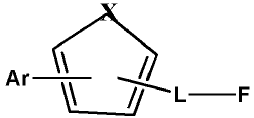
wherein X represents Se or Te;
wherein Ar is an aromatic system selected from the list consisting of 2-aminopyrimidine, 2-methylpyrimidine, pyrimidine, pyridine, purine and 7H-pyrrolo[2,3-d]pyrimidine; wherein the 5-membered Se or Te membered ring may additionally optionally incorporate one nitrogen atom;
wherein the 5-membered Se or Te containing ring may be optionally substituted by halogeno groups;
wherein the excitation spectrum of the fluorescence acceptor dye F overlaps with the emission spectrum of the luminescent donor P.

20. A probe according to claim 19 wherein the fragment P-L of the probe is a bisubstrate inhibitor of the protein kinase.

21. A probe according to claim 19 wherein the 5-membered Se or Te containing ring is selenophene or tellurophene.

22. A probe according to claim 19 selected from ARC-1139 and ARC-1144.

## Patentansprüche

1. Bindungsassay-Methode für Proteinkinasen, wobei die Lumineszenz einer an eine Proteinkinase gebundenen photolumineszierenden Sonde gemessen wird, wobei die Sonde die Struktur P-L-F aufweist, wobei P ein Fragment enthält, das Lumineszenzdonoreigenschaften aufweist und spezifisch mit der purinbindenden Tasche der Ziel-Proteinkinase assoziiert, L ein Linkersystem ist und F ein Fluoreszenzakzeptorfarbstoff ist, wobei die Sonde ein auf einer Thiophen-, Selenophen- oder Tellurophen-Gruppierung basierendes heteroaromatisches Fragment enthält, das mit einem aromatischen System Ar verknüpft ist und über ein Linkersystem L mit einem Fluoreszenzfarbstoff F verbunden ist, wobei die Sonde die folgende Struktur aufweist: 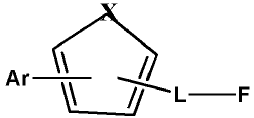
wobei X für S, Se oder Te steht;
wobei Ar ein aromatisches System aus der Liste bestehend aus 2-Aminopyrimidin, 2-Methylpyrimidin, Pyrimidin, Pyridin, Purin und 7H-Pyrrolo[2,3-d]pyrimidin ist;
wobei der 5-gliedrige S, Se oder Te enthaltende Ring gegebenenfalls zusätzlich ein Stickstoffatom enthalten kann;
wobei der 5-gliedrige S, Se oder Te enthaltende Ring gegebenenfalls durch Halogengruppen substituiert sein kann;
wobei das Anregungsspektrum des Fluoreszenzakzeptorfarbstoffs F mit dem Emissionsspektrum des Lumineszenzdonors P überlappt;
umfassend (a) das Anregen der an die purinbindende Tasche der Ziel-Proteinkinase gebundenen Sonde mit einem Einphotonenanregungspuls im Wellenlängenbereich von 280-450 nm und (b) das Messen der von der Sonde emittierten Lumineszenzstrahlung im Wellenlängenbereich von 450-1000 nm im Zeitverzögerungsmodus;
wobei die Zeitverzögerung so beschaffen ist, dass die von der Sonde emittierte Lumineszenzstrahlung frei von durch organische Verbindungen und Lichtstreuung verursachter Hintergrund-Fluoreszenz mit einer Lebensdauer von 1 bis 10 Nanosekunden ist.

2. In-vitro-Bindungsassay-Methode für Proteinkinase in lebenden Zellen, wobei die Lumineszenz einer photolumineszierenden Sonde mit einem Fluoreszenzmikroskop, einem Fluoreszenzspektrometer oder einem Fluoreszenzplattenlesegerät detektiert wird, wobei die Sonde die Struktur P-L-F aufweist, wobei P ein Fragment enthält, das Lumineszenzdonoreigenschaften aufweist und spezifisch mit der purinbindenden Tasche der Ziel-Proteinkinase assoziiert, L ein Linkersystem ist und F ein Fluoreszenzakzeptorfarbstoff ist, wobei die Sonde ein auf einer Thiophen-, Selenophen- oder Tellurophen-Gruppierung basierendes heteroaromatisches Fragment enthält, das mit einem aromatischen System Ar verknüpft ist und über ein Linkersystem L mit einem Fluoreszenzfarbstoff F verbunden ist, wobei die Sonde die folgende Struktur aufweist: 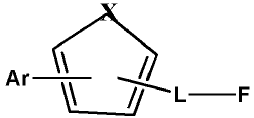
wobei X für S, Se oder Te steht;
wobei Ar ein aromatisches System aus der Liste bestehend aus 2-Aminopyrimidin, 2-Methylpyrimidin, Pyrimidin, Pyridin, Purin und 7H-Pyrrolo[2,3-d]pyrimidin ist;
wobei L für 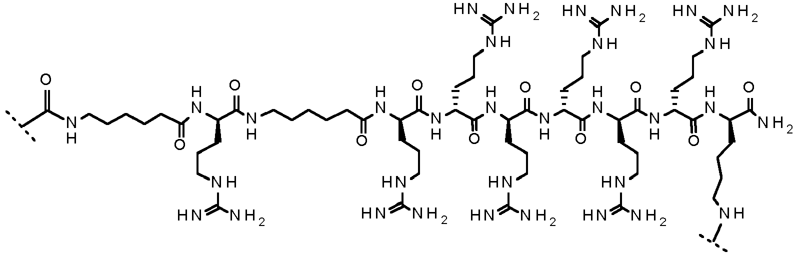 steht;
wobei der 5-gliedrige S, Se oder Te enthaltende Ring gegebenenfalls zusätzlich ein Stickstoffatom enthalten kann;
wobei der 5-gliedrige S, Se oder Te enthaltende Ring gegebenenfalls durch Halogengruppen substituiert sein kann;
wobei das Anregungsspektrum des Fluoreszenzakzeptorfarbstoffs F mit dem Emissionsspektrum des Lumineszenzdonors P überlappt;
umfassend (a) das Anregen der an die purinbindende Tasche der Ziel-Proteinkinase gebundenen Sonde mit einem Einphotonenanregungspuls im Wellenlängenbereich von 280-450 nm und (b) das zeitverzögerte Detektieren der von der Sonde emittierten Lumineszenzstrahlung im Wellenlängenbereich von 450-1000 nm;
wobei die Zeitverzögerung so beschaffen ist, dass die von der Sonde emittierte Lumineszenzstrahlung frei von durch organische Verbindungen und Lichtstreuung verursachter Hintergrund-Fluoreszenz mit einer Lebensdauer von 1 bis 10 Nanosekunden ist.

3. Bindungsassay-Methode nach Anspruch 1, wobei es sich bei dem Fragment P-L der Sonde um einen Bisubstrat-Inhibitor der Proteinkinase handelt.

4. Verwendung einer photolumineszierenden Sonde, die die Struktur P-L-F aufweist, wobei P ein Fragment enthält, das Lumineszenzdonoreigenschaften aufweist und spezifisch mit der purinbindenden Tasche der Ziel-Proteinkinase assoziiert, L ein Linkersystem ist und F ein Fluoreszenzakzeptorfarbstoff ist, wobei die Sonde ein auf einer Thiophen-, Selenophen- oder Tellurophen-Gruppierung basierendes heteroaromatisches Fragment enthält, das mit einem aromatischen System Ar verknüpft ist und über ein Linkersystem L mit einem Fluoreszenzfarbstoff F verbunden ist, wobei die Sonde die folgende Struktur aufweist: 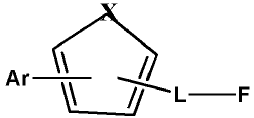
wobei X für S, Se oder Te steht;
wobei Ar ein aromatisches System aus der Liste bestehend aus 2-Aminopyrimidin, 2-Methylpyrimidin, Pyrimidin, Pyridin, Purin und 7H-Pyrrolo[2,3-d]pyrimidin ist;
wobei der 5-gliedrige S, Se oder Te enthaltende Ring gegebenenfalls zusätzlich ein Stickstoffatom enthalten kann;
wobei der 5-gliedrige S, Se oder Te enthaltende Ring gegebenenfalls durch Halogengruppen substituiert sein kann;
wobei das Anregungsspektrum des Fluoreszenzakzeptorfarbstoffs F mit dem Emissionsspektrum des Lumineszenzdonors P überlappt;
bei einem Bindungsassay für Proteinkinasen;
wobei bei dem Bindungsassay die Lumineszenz einer photolumineszierenden Sonde im Zeitverzögerungsmodus gemessen wird;
wobei die Sonde, wenn sie an die purinbindende Tasche der Ziel-Proteinkinase gebunden ist, mit einem Einphotonenanregungspuls im Wellenlängenbereich von 280-450 nm angeregt wird und die Sonde Lumineszenzstrahlung im Wellenlängenbereich von 450-1000 nm emittiert; wobei die Zeitverzögerung so beschaffen ist, dass die von der Sonde emittierte Lumineszenzstrahlung frei von durch organische Verbindungen und Lichtstreuung verursachter Hintergrund-Fluoreszenz mit einer Lebensdauer von 1 bis 10 Nanosekunden ist.

5. Verwendung eines Bindungsassays nach Anspruch 1 oder Anspruch 3 zur quantitativen Bestimmung einer Proteinkinase.

6. Verwendung eines Bindungsassays nach Anspruch 1 oder Anspruch 3 zur Bestimmung der Affinität eines Proteinkinaseinhibitors unter Verwendung der Verdrängung der Sonde aus einem Komplex der Sonde mit der Proteinkinase durch einen kompetitiven Inhibitor.

7. Verwendung eines Bindungsassays nach Anspruch 1 oder Anspruch 3 zur Charakterisierung der Dissoziationskinetik eines Komplexes eines Inhibitors mit einer Proteinkinase.

8. Verwendung eines Bindungsassays nach Anspruch 1 oder Anspruch 3 zur quantitativen Bestimmung der Aktivität einer Proteinkinase in einer Körperflüssigkeit, einem Zelllysat oder einem Gewebeextrakt.

9. Verwendung eines Bindungsassays nach Anspruch 1 oder Anspruch 3 zur Überwachung der Rezeptoraktivierung und zellulären Konzentration von cAMP durch Analyse eines Zelllysats.

10. Verwendung eines Bindungsassays nach Anspruch 1 oder Anspruch 3 zur quantitativen Bestimmung einer Proteinkinase als Biomarker für eine Erkrankung in einer Körperflüssigkeit, einem Zelllysat oder einem Gewebeextrakt.

11. Verwendung eines Bindungsassays nach Anspruch 2 zur Charakterisierung eines Inhibitors einer Proteinkinase in lebenden Zellen.

12. Verwendung eines Bindungsassays nach Anspruch 2 zur Charakterisierung von Agonisten oder Antagonisten eines Gs- und Gi-proteingekoppelten Rezeptors durch Messung der durch Aktivierung von PKA durch den sekundären Botenstoff cAMP verursachten Änderung der Lumineszenzintensität der Sonde in lebenden Zellen.

13. Verwendung eines Bindungsassays nach Anspruch 2 zur Charakterisierung von Agonisten oder Antagonisten eines metabotropen oder ionotropen Rezeptors durch Messung der durch Aktivierung von anderen Proteinkinasen als PKA verursachten Änderung der Lumineszenz der Sonde in lebenden Zellen.

14. Verwendung einer photolumineszierenden Sonde, die die Struktur P-L-F aufweist, wobei P ein Fragment enthält, das Lumineszenzdonoreigenschaften aufweist und spezifisch mit der purinbindenden Tasche einer Ziel-Proteinkinase assoziiert, L ein Linkersystem ist und F ein Fluoreszenzakzeptorfarbstoff ist, wobei die Sonde ein auf einer Thiophen-, Selenophen- oder Tellurophen-Gruppierung basierendes heteroaromatisches Fragment enthält, das mit einem aromatischen System Ar verknüpft ist und über ein Linkersystem L mit einem Fluoreszenzfarbstoff F verbunden ist, wobei die Sonde die folgende Struktur aufweist: 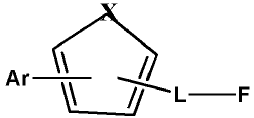
wobei X für S, Se oder Te steht;
wobei Ar ein aromatisches System aus der Liste bestehend aus 2-Aminopyrimidin, 2-Methylpyrimidin, Pyrimidin, Pyridin, Purin und 7H-Pyrrolo[2,3-d]pyrimidin ist;
wobei der 5-gliedrige S, Se oder Te enthaltende Ring gegebenenfalls zusätzlich ein Stickstoffatom enthalten kann;
wobei der 5-gliedrige S, Se oder Te enthaltende Ring gegebenenfalls durch Halogengruppen substituiert sein kann;
wobei das Anregungsspektrum des Fluoreszenzakzeptorfarbstoffs F mit dem Emissionsspektrum des Lumineszenzdonors P überlappt;
wobei es sich bei der Ziel-Proteinkinase um PKAc handelt;
bei einer Hochdurchsatz-Analysenmethode zur Ermittlung des Niveaus der PKAc-Aktivität in einer Blutplasmaprobe;
wobei bei der Methode die Lumineszenz einer photolumineszierenden Sonde im Zeitverzögerungsmodus gemessen wird;
wobei die Sonde, wenn sie an die purinbindende Tasche der Ziel-Proteinkinase gebunden ist, mit einem Einphotonenanregungspuls im Wellenlängenbereich von 280-450 nm angeregt wird und die Sonde Lumineszenzstrahlung im Wellenlängenbereich von 450-1000 nm emittiert; wobei die Zeitverzögerung so beschaffen ist, dass die von der Sonde emittierte Lumineszenzstrahlung frei von durch organische Verbindungen und Lichtstreuung verursachter Hintergrund-Fluoreszenz mit einer Lebensdauer von 1 bis 10 Nanosekunden ist.

15. Methode oder Verwendung nach einem der Ansprüche 1 bis 14, wobei es sich bei dem 5-gliedrigen S, Se oder Te enthaltenden Ring um Thiophen, Selenophen oder Tellurophen handelt.

16. Methode oder Verwendung nach einem der Ansprüche 1 bis 14, wobei die Sonde aus ARC-669, ARC-676, ARC-679, ARC-1063, ARC-1122, ARC-1128, ARC-1130, ARC-1133, ARC-1136, ARC-1139 und ARC-1144 ausgewählt ist.

17. Verwendung einer photolumineszierenden Sonde, die die Struktur P-L aufweist, wobei P ein Fragment enthält, das Lumineszenzdonoreigenschaften aufweist und spezifisch mit der purinbindenden Tasche einer Ziel-Proteinkinase assoziiert, und L ein Linkersystem ist; wobei die Sonde aus ARC-668, ARC-1121, ARC-1123, ARC-1129, ARC-1132, ARC-1135 und ARC-1138 ausgewählt ist; wobei es sich bei der Ziel-Proteinkinase um eine mit einem Fluoreszenzfarbstoff F markierte Proteinkinase handelt;
wobei das Anregungsspektrum des Fluoreszenzfarbstoffs F mit dem Emissionsspektrum von P überlappt;
bei einer In-vitro-Methode zur Überwachung der Kinaseaktivität in lebenden Zellen;
wobei die Lumineszenz einer photolumineszierenden Sonde im Zeitverzögerungsmodus gemessen wird;
wobei die Sonde, wenn sie an die purinbindende Tasche der Ziel-Proteinkinase gebunden ist, mit einem Einphotonenanregungspuls im Wellenlängenbereich von 280-450 nm angeregt wird und die Sonde Lumineszenzstrahlung im Wellenlängenbereich von 450-1000 nm emittiert;
wobei die Zeitverzögerung so beschaffen ist, dass die von der Sonde emittierte Lumineszenzstrahlung frei von durch organische Verbindungen und Lichtstreuung verursachter Hintergrund-Fluoreszenz mit einer Lebensdauer von 1 bis 10 Nanosekunden ist.

18. Methode oder Verwendung nach einem der Ansprüche 1, 3 bis 10, 14 oder 15, wobei L für: 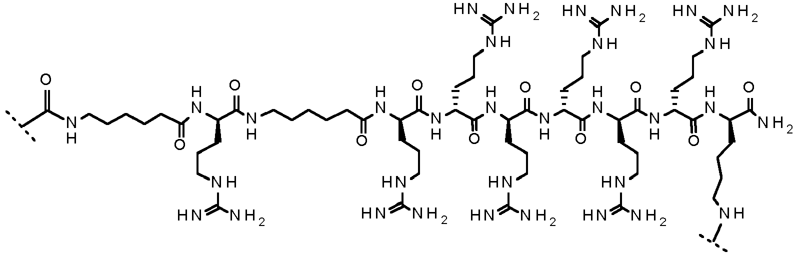 steht.

19. Photolumineszierende Sonde, die die Struktur P-L-F aufweist, wobei P ein Fragment enthält, das Lumineszenzdonoreigenschaften aufweist und spezifisch mit der purinbindenden Tasche der Ziel-Proteinkinase assoziiert, L ein Linkersystem ist und F ein Fluoreszenzakzeptorfarbstoff ist, wobei die Sonde ein auf einer Selenophen- oder Tellurophen-Gruppierung basierendes heteroaromatisches Fragment enthält, das mit einem aromatischen System Ar verknüpft ist und über ein Linkersystem L mit einem Fluoreszenzfarbstoff F verbunden ist, wobei die Sonde die folgende Struktur aufweist: 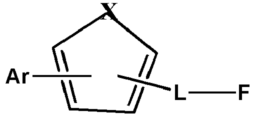
wobei X für Se oder Te steht;
wobei Ar ein aromatisches System aus der Liste bestehend aus 2-Aminopyrimidin, 2-Methylpyrimidin, Pyrimidin, Pyridin, Purin und 7H-Pyrrolo[2,3-d]pyrimidin ist;
wobei der 5-gliedrige Se oder Te enthaltende Ring gegebenenfalls zusätzlich ein Stickstoffatom enthalten kann;
wobei der 5-gliedrige Se oder Te enthaltende Ring gegebenenfalls durch Halogengruppen substituiert sein kann;
wobei das Anregungsspektrum des Fluoreszenzakzeptorfarbstoffs F mit dem Emissionsspektrum des Lumineszenzdonors P überlappt.

20. Sonde nach Anspruch 19, wobei es sich bei dem Fragment P-L der Sonde um einen Bisubstrat-Inhibitor der Proteinkinase handelt.

21. Sonde nach Anspruch 19, wobei es sich bei dem 5-gliedrigen Se oder Te enthaltenden Ring um Selenophen oder Tellurophen handelt.

22. Sonde nach Anspruch 19, ausgewählt aus ARC-1139 und ARC-1144.

## Revendications

1. Procédé de dosage par liaison pour protéines kinases dans lequel la luminescence d'une sonde photoluminescente liée à une protéine kinase est mesurée, la sonde ayant la structure P-L-F dans laquelle P contient un fragment qui a des propriétés de donneur de luminescence et qui s'associe spécifiquement avec la poche de fixation de la purine de la protéine kinase cible, L est un système de liaison et F est un colorant accepteur de fluorescence, ladite sonde contenant un fragment hétéroaromatique basé sur une fraction thiophène, sélénophène ou tellurophène liée à un système aromatique Ar et relié par un système de liaison L à un colorant fluorescent F, ladite sonde ayant la structure suivante : 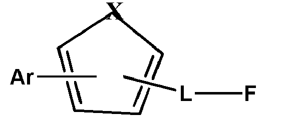
dans laquelle X représente S, Se ou Te ;
dans laquelle Ar est un système aromatique choisi dans la liste constituée par la 2-aminopyrimidine, la 2-méthylpyrimidine, la pyrimidine, la pyridine, la purine et la 7H-pyrrolo[2,3-d]pyrimidine ;
dans laquelle le cycle à 5 chaînons à maillon S, Se ou Te peut en plus éventuellement incorporer un atome d'azote ;
dans laquelle le cycle à 5 chaînons contenant S, Se ou Te peut éventuellement être substitué par des groupes halogéno ;
dans laquelle le spectre d'excitation du colorant accepteur de fluorescence F chevauche le spectre d'émission du donneur luminescent P ;
comprenant (a) l'excitation de ladite sonde liée à la poche de fixation de la purine de la protéine kinase cible avec une impulsion d'excitation monophotonique dans la gamme de longueurs d'onde 280-450 nm et (b) la mesure en mode temporisé du rayonnement de luminescence émis par la sonde dans la gamme de longueurs d'onde 450-1000 nm ; la temporisation étant telle que le rayonnement de luminescence émis par la sonde est mesuré sans la fluorescence de fond d'une durée de vie de 1 à 10 nanosecondes induite par les composés organiques et la diffusion de la lumière.

2. Procédé de dosage par liaison in vitro pour protéines kinases dans des cellules vivantes dans lequel la luminescence d'une sonde photoluminescente est détectée à l'aide d'un microscope de fluorescence, d'un spectromètre de fluorescence ou d'un lecteur de plaques en fluorescence, la sonde ayant la structure P-L-F dans laquelle P contient un fragment qui a des propriétés de donneur de luminescence et qui s'associe spécifiquement avec la poche de fixation de la purine de la protéine kinase cible, L est un système de liaison et F est un colorant accepteur de fluorescence, ladite sonde contenant un fragment hétéroaromatique basé sur une fraction thiophène, sélénophène ou tellurophène liée à un système aromatique Ar et relié par un système de liaison L à un colorant fluorescent F, ladite sonde ayant la structure suivante : 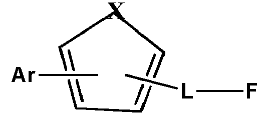
dans laquelle X représente S, Se ou Te ;
dans laquelle Ar est un système aromatique choisi dans la liste constituée par la 2-aminopyrimidine, la 2-méthylpyrimidine, la pyrimidine, la pyridine, la purine et la 7H-pyrrolo[2,3-d]pyrimidine ;
dans laquelle L représente 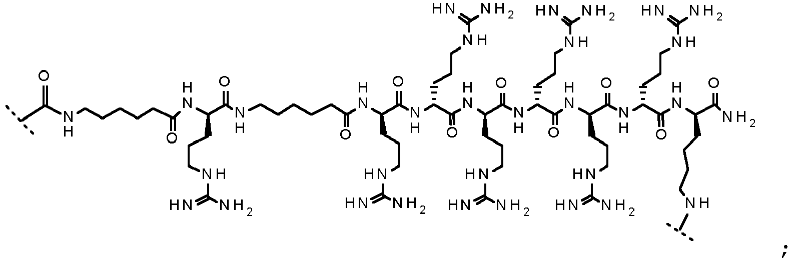
dans laquelle le cycle à 5 chaînons à maillon S, Se ou Te peut en plus éventuellement incorporer un atome d'azote ;
dans laquelle le cycle à 5 chaînons contenant S, Se ou Te peut éventuellement être substitué par des groupes halogéno ;
dans laquelle le spectre d'excitation du colorant accepteur de fluorescence F chevauche le spectre d'émission du donneur luminescent P ;
comprenant (a) l'excitation de ladite sonde liée à la poche de fixation de la purine de la protéine kinase cible avec une impulsion d'excitation monophotonique dans la gamme de longueurs d'onde 280-450 nm et (b) la détection temporisée du rayonnement de luminescence émis par la sonde dans la gamme de longueurs d'onde 450-1000 nm ;
la temporisation étant telle que le rayonnement de luminescence émis par la sonde est mesuré sans la fluorescence de fond d'une durée de vie de 1 à 10 nanosecondes induite par les composés organiques et la diffusion de la lumière.

3. Procédé de dosage par liaison selon la revendication 1 dans lequel le fragment P-L de la sonde est un inhibiteur bisubstrat de la protéine kinases.

4. Utilisation d'une sonde photoluminescente qui a la structure P-L-F dans laquelle P contient un fragment qui a des propriétés de donneur de luminescence et qui s'associe spécifiquement avec la poche de fixation de la purine d'une protéine kinase cible, L est un système de liaison et F est un colorant accepteur de fluorescence, ladite sonde contenant un fragment hétéroaromatique basé sur une fraction thiophène, sélénophène ou tellurophène liée à un système aromatique Ar et relié par un système de liaison L à un colorant fluorescent F, ladite sonde ayant la structure suivante : 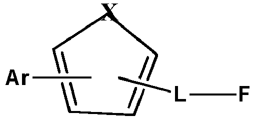
dans laquelle X représente S, Se ou Te ;
dans laquelle Ar est un système aromatique choisi dans la liste constituée par la 2-aminopyrimidine, la 2-méthylpyrimidine, la pyrimidine, la pyridine, la purine et la 7H-pyrrolo[2,3-d]pyrimidine ;
dans laquelle le cycle à 5 chaînons à maillon S, Se ou Te peut en plus éventuellement incorporer un atome d'azote ;
dans laquelle le cycle à 5 chaînons contenant S, Se ou Te peut éventuellement être substitué par des groupes halogéno ;
dans laquelle le spectre d'excitation du colorant accepteur de fluorescence F chevauche le spectre d'émission du donneur luminescent P ;
dans un dosage par liaison pour protéines kinases ;
dans laquelle, dans le dosage par liaison, la luminescence d'une sonde photoluminescente est mesurée en mode temporisé ;
dans laquelle ladite sonde, lorsqu'elle est liée à la poche de fixation de la purine de la protéine kinase cible, est excitée avec une impulsion d'excitation monophotonique dans la gamme de longueurs d'onde 280-450 nm et la sonde émet un rayonnement de luminescence dans la gamme de longueurs d'onde 450-1000 nm ;
la temporisation étant telle que le rayonnement de luminescence émis par la sonde est mesuré sans la fluorescence de fond d'une durée de vie de 1 à 10 nanosecondes induite par les composés organiques et la diffusion de la lumière.

5. Utilisation d'un dosage par liaison selon la revendication 1 ou la revendication 3 pour la quantification d'une protéine kinase.

6. Utilisation d'un dosage par liaison selon la revendication 1 ou la revendication 3 pour la détermination de l'affinité d'un inhibiteur de protéines kinases à l'aide du déplacement de la sonde d'un complexe de la sonde avec ladite protéine kinase par un inhibiteur compétitif.

7. Utilisation d'un dosage par liaison selon la revendication 1 ou la revendication 3 pour la caractérisation de la cinétique de dissociation d'un complexe d'un inhibiteur avec une protéine kinase.

8. Utilisation d'un dosage par liaison selon la revendication 1 ou la revendication 3 pour la quantification de l'activité d'une protéine kinase dans un fluide corporel, un lysat cellulaire ou un extrait tissulaire.

9. Utilisation d'un dosage par liaison selon la revendication 1 ou la revendication 3 pour la surveillance de l'activation des récepteurs et de la concentration cellulaire de l'AMPc par analyse d'un lysat cellulaire.

10. Utilisation d'un dosage par liaison selon la revendication 1 ou la revendication 3 pour la quantification d'une protéine kinase servant de biomarqueur d'une maladie dans un fluide corporel, une cellule sanguine ou un extrait tissulaire.

11. Utilisation d'un dosage par liaison selon la revendication 2 pour la caractérisation d'un inhibiteur d'une protéine kinase dans des cellules vivantes.

12. Utilisation d'un dosage par liaison selon la revendication 2 pour la caractérisation d'un agoniste ou antagoniste d'un récepteur couplé à la protéine Gs ou Gi par mesure du changement d'intensité de luminescence de la sonde provoqué par l'activation des PKA par l'AMPc second messager dans des cellules vivantes.

13. Utilisation d'un dosage par liaison selon la revendication 2 pour la caractérisation d'un agoniste ou antagoniste d'un récepteur métabotropiquee ou ionotropique par mesure du changement de luminescence de la sonde provoqué par l'activation d'autres protéines kinases que les PKA dans des cellules vivantes.

14. Utilisation d'une sonde photoluminescente qui a la structure P-L-F dans laquelle P contient un fragment qui a des propriétés de donneur de luminescence et qui s'associe spécifiquement avec la poche de fixation de la purine d'une protéine kinase cible, L est un système de liaison et F est un colorant accepteur de fluorescence, ladite sonde contenant un fragment hétéroaromatique basé sur une fraction thiophène, sélénophène ou tellurophène liée à un système aromatique Ar et relié par un système de liaison L à un colorant fluorescent F, ladite sonde ayant la structure suivante : 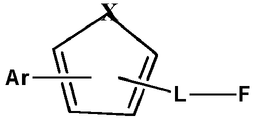
dans laquelle X représente S, Se ou Te ;
dans laquelle Ar est un système aromatique choisi dans la liste constituée par la 2-aminopyrimidine, la 2-méthylpyrimidine, la pyrimidine, la pyridine, la purine et la 7H-pyrrolo[2,3-d]pyrimidine ;
dans laquelle le cycle à 5 chaînons à maillon S, Se ou Te peut en plus éventuellement incorporer un atome d'azote ;
dans laquelle le cycle à 5 chaînons contenant S, Se ou Te peut éventuellement être substitué par des groupes halogéno ;
dans laquelle le spectre d'excitation du colorant accepteur de fluorescence F chevauche le spectre d'émission du donneur luminescent P ;
dans laquelle la protéine kinase cible est la PKAc ; dans un procédé d'analyse à haut débit pour l'établissement du niveau d'activité de la PKAc dans un échantillon de plasma sanguin ;
dans laquelle, dans le procédé, la luminescence d'une sonde photoluminescente est mesurée en mode temporisé ; dans laquelle ladite sonde, lorsqu'elle est liée à la poche de fixation de la purine de la protéine kinase cible, est excitée avec une impulsion d'excitation monophotonique dans la gamme de longueurs d'onde 280-450 nm et la sonde émet un rayonnement de luminescence dans la gamme de longueurs d'onde 450-1000 nm ;
la temporisation étant telle que le rayonnement de luminescence émis par la sonde est mesuré sans la fluorescence de fond d'une durée de vie de 1 à 10 nanosecondes induite par les composés organiques et la diffusion de la lumière.

15. Procédé ou utilisation selon l'une quelconque des revendications 1 à 14 dans lesquels le cycle à 5 chaînons à maillon S, Se ou Te est le thiophène, le sélénophène ou le tellurophène.

16. Procédé ou utilisation selon l'une quelconque des revendications 1 à 14 dans lesquels la sonde est choisie parmi ARC-669, ARC-676, ARC-679, ARC-1063, ARC-1122, ARC-1128, ARC-1130, ARC-1133, ARC-1136, ARC-1139 et ARC-1144.

17. Utilisation d'une sonde photoluminescente qui a la structure P-L dans laquelle P contient un fragment qui a des propriétés de donneur de luminescence et qui s'associe spécifiquement avec la poche de fixation de la purine d'une protéine kinase cible et L est un système de liaison ; ladite sonde étant choisie parmi ARC-668, ARC-1121, ARC-1123, ARC-1129, ARC-1132, ARC-1135 et ARC-1138 ; la protéine kinase cible étant une protéine kinase marquée avec un colorant fluorescent F ;
dans laquelle le spectre d'excitation du colorant fluorescent F chevauche le spectre d'émission de P ; dans un procédé in vitro destiné à surveiller l'activité kinase dans des cellules vivantes ;
dans laquelle la luminescence d'une sonde photoluminescente est mesurée en mode temporisé ;
dans laquelle ladite sonde, lorsqu'elle est liée à la poche de fixation de la purine de la protéine kinase cible, est excitée avec une impulsion d'excitation monophotonique dans la gamme de longueurs d'onde 280-450 nm et la sonde émet un rayonnement de luminescence dans la gamme de longueurs d'onde 450-1000 nm ;
la temporisation étant telle que le rayonnement de luminescence émis par la sonde est mesuré sans la fluorescence de fond d'une durée de vie de 1 à 10 nanosecondes induite par les composés organiques et la diffusion de la lumière.

18. Procédé ou utilisation selon l'une quelconque des revendications 1, 3 à 10, 14 ou 15 dans lesquels L représente :

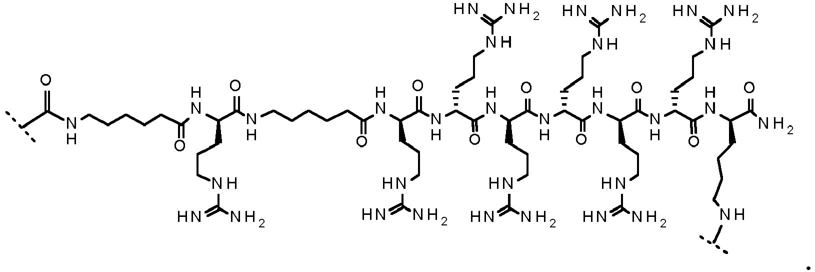

19. Sonde photoluminescente ayant la structure P-L-F dans laquelle P contient un fragment qui a des propriétés de donneur de luminescence et qui s'associe spécifiquement avec la poche de fixation de la purine de la protéine kinase cible, L est un système de liaison et F est un colorant accepteur de fluorescence, ladite sonde contenant un fragment hétéroaromatique basé sur une fraction sélénophène ou tellurophène liée à un système aromatique Ar et relié par un système de liaison L à un colorant fluorescent F, ladite sonde ayant la structure suivante : 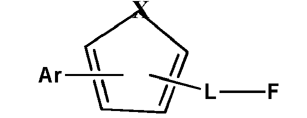
dans laquelle X représente Se ou Te ;
dans laquelle Ar est un système aromatique choisi dans la liste constituée par la 2-aminopyrimidine, la 2-méthylpyrimidine, la pyrimidine, la pyridine, la purine et la 7H-pyrrolo[2,3-d]pyrimidine ;
dans laquelle le cycle à 5 chaînons à maillon Se ou Te peut en plus éventuellement incorporer un atome d'azote ; dans laquelle le cycle à 5 chaînons contenant Se ou Te peut éventuellement être substitué par des groupes halogéno ;
dans laquelle le spectre d'excitation du colorant accepteur de fluorescence F chevauche le spectre d'émission du donneur luminescent P.

20. Sonde selon la revendication 19, le fragment P-L de la sonde étant un inhibiteur bisubstrat de la protéine kinase.

21. Sonde selon la revendication 19 dans laquelle le cycle à 5 chaînons contenant Se ou Te est le sélénophène ou le tellurophène.

22. Sonde selon la revendication 19 choisie entre ARC-1139 et ARC-1144.
